# EUROPEAN PATENT APPLICATION

(11) **EP 2 518 157 A1**
(43) Date of publication of application: **31.10.2012**
(21) Application number: 11305488.6
(22) Date of filing: 26.04.2011
(51) Int. Cl.: C12Q 1/48, G01N 33/50

(54) **Test Systems and methods for identifying a compound altering cellular DDR activity**

(71) Applicant: SANOFI, 75008 Paris (FR)
(72) Inventor: The designation of the inventor has not yet been filed
(74) Representative: Schneider, Rudolf

(57) **Abstract**

The present invention relates to test systems and methods for identifying a compound altering cellular Discoidin domain receptor tyrosine kinase activity.

## Description

The present invention relates to test systems and methods for identifying a compound altering cellular DDR (Discoidin domain receptor) tyrosine kinase activity.

Discoidin domain receptors (DDRs) are non-integrin type receptors for collagen. The two mammalian gene products, discoidin domain receptors 1 and 2 (DDR1 and DDR2), constitute a sub-family of tyrosine kinase receptors. They are selectively expressed in a number of different cell types in organs. When the DDRs are activated by collagen, the DDRs regulate cell adhesion, proliferation and extracellular matrix remodeling. DDR1 can form ligand-independent homodimers at a cell surface already in the absence of collagen. Collagen binding to DDR1 dimers induces their internalization and subsequent activation through tyrosine auto-/transphosphorylation. Collagen activation of DDR1 is following a delayed time course, and maximum stimulation is usually observed after 90 minutes to 16 hours dependent on cell type and on the collagen applied. Receptor internalization finally results in phosphorylation and subsequent sequence transduction involving signaling cascade molecules like ShcA, Shp-2, Nck2, the transcription factors NF-κb, Stat5, and the kinases p38, P13K, Pyk2, JNK and cJun. Finally, the signaling cascade affects differentiation, ECM remodeling, migration and cell cycle control. DDR molecules are believed to play a role in various indications and diseases like, for example, cancer, including tumor invasion and metastasis, atherosclerosis, including atherosclerotic plug formation, fibrosis, in particular fibrosis of kidney, liver, lungs and skin, diabetes, in particular diabetic nephropathy, and in inflammation and arthritis. There is, therefore, a need for test systems and assay methods for the identification of modulators of the DDR signaling pathways. In particular, there is a need for the identification of inhibitors of the DDR pathways. In order to meet the requirements of clinical early research, there is also a need for reliable mid-to-high throughput assay systems which allow the identification of modulators which affect either the kinase functions of a DDR or/and activation of a DDR through collagen.

KR2009042397 discloses an assay system using transgenic insect cell, wherein the binding of the cells to collagen bound to a surface is measured. KR2009042398 discloses Actinomycin D as inhibitor of the binding of DDR to collagen. WO2007/044894 discloses cell-free translation/transcription systems. WO2003/082328 discloses an assay for DDR wherein the maturation of dendritic cells is used as readout. WO 98/45711 discloses assay for DDR1 and DDR2 activity. The phospho-tyrosine content was measured by lysing cells, immunoprecipitation and subsequent immunoblotting. WO98/34954 discloses assays for DDR1 involving the PTB domain. WO95/14089 discloses MCK-10 and the truncated form CCK-2. It is also disclosed that sequences can be used in a general manner in assay systems. WO95/02187 disclose in a very general manner that cell-based assays can be used for protein tyrosine kinase of SEQ ID No. 1 in WO95/02187. Vogel W.F. et al. ((2006), Cellular Signalling, 18:1108-1116) and Vogel W. F. ((1999), FASEB J., 13(S):S77-S82) give an overview on DDR polypeptides in general. Vogel W. et al. ((1997), Mol Cell. 1(1):13-23) discloses that discoidin domain receptor tyrosine kinases are activated by collagen. Vogel W. et al. ((2000), J. Biol. Chem., 275(8):5779-5784) discloses that Discoidin domain receptor 1 is activated independently of β1-integrin. Bantscheff M. et al. ((2007), Nat Biotechnol. 25(9):1035-44) inter alia discloses that receptor tyrosine kinase DDR1 is target of the tyrosine kinase inhibitor imatinib.

Discoidin Domain Receptor (DDR) according to the present invention is a polypeptide of the subfamily of receptor tyrosine kinases which possesses an extracellular domain related to the lectin discoidin, found in the slime mold *Dictyostelium discoideum.* All members of the subfamily share the approximately 160-amino acid-long amino terminal discoidin homology domain followed by a single transmembrane region, and extended juxtamembrane region, and a catalytic tyrosine kinase domain.

Two receptor tyrosine kinase genes with discoidin homology have been cloned in humans and are known as discoidin domain receptor DDR1 and DDR2.

DDR1 appears in at least five isoforms:
DDR1a (876AA): NM_001954
   (http://www.ncbi.nlm.nih.gov/entrez/viewer.fcgi?val=NM_001954; cDNA: SEQ ID No. 1; protein: SEQ ID No. 2),
DDR1b (913AA): NM_013993
   (http://www.ncbi.nlm.nih.gov/entrez/viewer.fcgi?val=NM_013993; cDNA: SEQ ID No. 3; protein: SEQ ID No. 4),
DDR1c (919AA): NM_013994
   (http://www.ncbi.nlm.nih.gov/entrez/viewer.fcgi?val=NM_013994; cDNA: SEQ ID No. 5; protein: SEQ ID No. 6), and
DDR1 d (Accession No. AF353182), and DDR1 e (Accession No. AF353183), which are generated by alternative splicing.
DDR1 a and DDR1 b receptor isoforms lack 37 or 6 amino acids in the juxtamembrane or kinase domain respectively.
DDR1 d and DDR1 e are truncated variants lacking either the entire kinase region or parts of the juxtamembrane region and the ATP binding site (Alves et al., (2001), FASEB J., 15(7): 1321).
For DDR2, two splice isoform exist, namely NM_006182.2
   (http://www.ncbi.nlm.nih.gov/nuccore/NM-006182.2) and NM_001014796.1
   (http://www.ncbi.nlm.nih.gov/nuccore/NM-001014796.1; SEQ ID No. 7). The differences in the transcripts relate to the 5'UTR. The isoforms encode the same protein (SEQ ID No. 8).
DDR protein sequences are well conserved, including those from humans, worms and flies (Vogel, (2006), Cellular Signalling, 18: 1108-1116).
A murine DDR1 polypeptide is for example disclosed as DDR1 transcript variant 2 under NCBI Accession number NM_172962 (http://www.ncbi.nlm.nih.gov/nuccore/NM_172962; polypeptide: SEQ ID No. 10; cDNA: SEQ ID No. 9).

The present invention relates to a test system for determining Discoidin domain receptor DDR collagen-induced auto-/transphosphorylation activity comprising:
a) at least one cell containing at least one DDR polypeptide or functional derivative thereof localized in the plasma membrane of the cell,
b) either b1) or b2):
   b1) a binding partner capable of selectively recognizing phospho-tyrosine, which binding partner is a labeled binding partner,
   b2) a binding partner capable of selectively recognizing phospho-tyrosine and another binding partner, which is a labeled binding partner, and which is capable of selectively recognizing the binding partner recognizing phospho-tyrosine,
c) at least one reagent for fixing the cells on a solid support and permeabilizing the cells
d) at least one solid support for fixing the cells and for culturing the cells,
e) at least one collagen,
f) optionally at least one compound suspected of altering the collagen-induced auto-/transphosphorylation of the at least one DDR polypeptide or functional derivative thereof, and
g) means for detecting the binding partner of b) or c).

In another embodiment, the present invention relates to a method for identifying a compound altering DDR collagen-induced auto-/transphosphorylation activity, particular DDR1 a, DDR1 b, DDR1 c and/or DDR2 collagen-induced auto-/transphosphorylation activity, in particular collagen-induced auto-/transphosphorylation activity of one or more polypeptides having a sequence selected from SEQ ID No. 2, SEQ ID No. 4, SEQ ID No. 6, SEQ ID No. 8, or SEQ ID No. 10, comprising the steps of:
a) culturing at least one cell on a solid support,
   which cell contains at least one DDR polypeptide or functional derivative thereof localized in the plasma membrane of the cell,
b) adding to the cell culture of step a) at least one collagen, in particular wherein the collagen is selected from collagen type I, collagen type II, collagen type III or collagen type IV, or mixtures thereof,
c) incubating the cells of step b) in a cellular buffer,
d) fixing the cells of step c) on the solid support and permeabilizing the fixed cells, and optionally washing the cells,
e) adding at least one compound suspected of altering the collagen-induced auto-/transphosphorylation of the at least one DDR polypeptide or functional derivative thereof before step d),
f) carrying out step f1) or f2):
   f1) adding a binding partner to the cells of step d),
      which binding partner is capable of selectively recognizing phospho-tyrosine, and
      which binding partner is a labeled binding partner,
   f2)
      i) adding a binding partner to the cells of step d),
         which binding partner is capable of selectively recognizing phospho-tyrosine,
      ii) adding another binding partner to cells of step i),
      which another binding partner is a labeled binding partner, and
      which another binding partner is capable of selectively recognizing the binding partner capable of selectively recognizing phospho-tyrosine,
g) detecting the labeled binding partner.

Preferably, washing steps may be included where appropriate. A washing step may for example be included after fixing the permeabilizing the cells and before adding an anti-phospho-tyrosine antibody. In a preferred embodiment, washing of the cells is carried out after step a), step c), step d), step f1), step f2i) and/or step f2ii) of the method of the invention. The washing can be can be carried out using an appropriate buffer known to a skilled person, e.g. buffered saline solutions.

It is understood, that all embodiments below apply both to the test system and the method of the invention.

In a preferred embodiment, the DDR polypeptide is of mammalian origin. In a preferred embodiment, the DDR polypeptide is selected from DDR1a, DDR1b, DDR1c and DDR2. Such isoforms are for example known in humans and mice, as e.g. used in the examples.

In a more preferred embodiment, the DDR polypeptide is a human polypeptide and/or is selected from the polypeptides according to SEQ ID No. 2, SEQ ID No. 4, SEQ ID No. 6, SEQ ID No. 8, or SEQ ID No. 10, or combinations thereof. In further preferred embodiment, the DDR polypeptides lack the signal peptide. Such signal peptide may be present or may be deleted without significantly altering the DDR activity.

In another preferred embodiment, the DDR polypeptide is a murine polypeptide and/or is selected from the polypeptide according to SEQ ID No.10.
According to the present invention, human DDR proteins, including all isoforms and orthologs may be used. Preferred is the use of human DDR1a, human DDR1b, human DDR1c, and human DDR2.

In a particularly preferred embodiment, human DDR1a polypeptide according to SEQ ID No. 2 or encoded by SEQ ID No. 1, human DDR1b polypeptide according to SEQ ID No. 4 or encoded by SEQ ID No. 3, human DDR1c polypeptide according to SEQ ID No. 6 or encoded by SEQ ID No. 5 and/or human DDR2 polypeptide according to SEQ ID No. 8 or encoded by SEQ ID No. 7 are used. In further preferred embodiment, the DDR polypeptides lack the signal peptide. In yet another embodiment, combinations thereof may be used.

In another particularly preferred embodiment, a murine DDR1 polypeptide according to SEQ ID No. 10, or encoded by SEQ ID No. 9 is used.

Also, DDR proteins from other organisms, in particular mammalian organisms may be used. For example DDR1 a, DDR1 b, DDR1c from mouse, rat, or macaca, in particular mouse may be used. Mouse DDR1 isoforms are known and are e.g. described in NCBI accession Nos. NM_007584.2, NM_172962.1, NM_001198831.1, NM_001198833.1, and a mouse DDR2 polypeptide is disclosed by NCBI accession No. NM_022563.2.

As shown in the examples, the method of the invention can be carried out both with the various human DDR isoforms and with a murine DDR isoform. Thus, the method is very flexible. The test systems of the invention may contain these isoforms.

In another preferred embodiment, the DDR polypeptide is rat, mouse, fly, worm, slime mold or fish DDR polypeptide.

In addition, functional derivatives of the native DDR proteins may be used. The functional derivatives of a DDR polypeptide are understood as having at least 80% sequence identity, more preferably at least 90% sequence identity, even more preferred at least 95% sequence identity, most preferred at least 98% sequence identity to a *wildtype* DDR polypeptide, in particular to a DDR polypeptide having a sequence according to SEQ ID No. 2, SEQ ID No. 4, SEQ ID No. 6, or SEQ ID No. 8, and exhibiting substantially the same collagen-induced auto-/transphosphorylation activity as a *wildtype* DDR polypeptide. In a preferred embodiment, the substantially same collagen-induced auto-/transphosphorylation activity is understood as at least 10%, 20%, 30%, 50%, 70% or 90% of the collagen-induced auto-/transphosphorylation of a wildtype DDR polypeptide. The activity can be determined as explained in Example 1. In particular, the signal peptide may be missing. In a preferred embodiment, the functional derivative is a non-human homologue of DDR1 a, DDR1 b, DDR1 c or DDR2 from mouse, rat, macaca, cow, pig, horse, fish or dog.

According to the invention, at least one cell containing at least one DDR polypeptide or functional derivative thereof localized in the plasma membrane of the cell isused. Such localization can for example be determined by an immunofluorescence method using an antibody specifically recognizing the DDR polypeptide or functional derivative thereof.

Expression of a DDR polypeptide or functional derivative thereof can for example be determined by Western Blot analysis using an antibody specifically recognizing the DDR polypeptide or functional derivative thereof.

In a preferred embodiment, the cell is of mammalian origin. In a further preferred embodiment, the cell is a mammalian cell. In another embodiment, the cell allows stable expression of a DDR polypeptide or functional derivative thereof. In one embodiment, the at least one DDR polypeptide or functional derivative thereof are expressed as transgenes. In a preferred embodiment, the cells are produced recombinantly. Preferably, the cells are obtained by transfecting the cells with a suitable vector system.

Transfection with DNA yields stable as well as unstable (transient) cells or cell lines. Transient cell lines reflect the survival of the transfected DNA in extrachromosomal form; stable cell lines are understood to result from the integration into the genome resulting in the stable expression of the transfected gene. Cells can be transfected using any appropriate means, including viral vectors, chemical transfectants, electroporation, calcium phosphate co-precipitation and direct diffusion of DNA.

The cell may be isolated, maintained and cultured as known to the skilled person. Aside from temperature and gas mixture, the most commonly varied factor in cell culture systems is the growth medium. Recipes for growth media can vary in pH, glucose concentration, growth factor and the presence of other nutrient components among others. Growth factors used for supplement media are often derived from animal blood such as calf serum. Genetically modified cells may be obtained by inserting the full-length coding sequence of the component in question, as known to the skilled person. The skilled person in the art knows how to derive a nucleic acid sequence coding for the same and how to isolate or produce such a nucleic acid sequence using standard techniques of molecular biology. This can be accomplished, for example, by the use and combination of existing sequences using restriction enzymes. The nucleic acid may be combined with further elements, e.g., a promoter and a transcription start and stop signal and a translation start and stop signal in order to provide for expression of a DDR polypeptide. As used herein, vectors are agents that transport the transgene into the cell and may include appropriate transcriptional and translational control signals such as a promoter. Vectors can be plasmid, viral or others known in the art. The promoter can be inducible or constitutive, general or cell specific, nuclear or cytoplasmic specific promoter. Selection of promoters, vectors and other elements is a matter of routine design within the level of ordinary skill in the art. Many such elements are described in the literature and are available through commercial suppliers.

In the examples, the vector pcDNA5-FRT-TO_DEST was used, and Flp-In™-CHO cells (Invitrogen) were transfected. Thus, in a preferred embodiment, the cells are mammalian cells transfected with a polynucleotide encoding at least one DDR polypeptide or functional derivative thereof, particularly the cells are transfected CHO cells, most preferably transfected Flp-In^{™}-CHO cells.

In a particularly preferred embodiment, a mammalian cell, more preferably CHO cell, most preferably a Flp-In^{™}-CHO cell is used as parental cell. The Flp-In^{™}-CHO cells contain a single stably integrated FRT site at a transcriptionally active locus. The Flp-In^{™}-CHO cell was created by transfecting CHO cells with pFRT/lacZeo2 and selecting for Zeocin^{™}-(Invitrogen) resistant clones. Co-transfection of Flp-In^{™} expression vector and the Flp recombinase vector pOG44 results in targeted integration of the expression vector to the same locus in every cell.

In a preferred embodiment, the parental cells do not contain DDR polypeptides localized in the plasma membrane of the cell. As shown in Example 1, Flp-In^{™}-CHO cell do not show significant DDR expression, as determined by Western Blot analysis.

Optionally, the cell may be part of a tissue; however, the method of the invention is preferably an ex *vivo* method. In a preferred embodiment of the invention the cell is from a cell line (many of which are well characterized and provide for constant conditions and convenient handling), particularly a mammalian cell line, more particularly a human or mouse cell line. Examples of suitable cell lines are included but are not limited to HEK 293, 745-A, A-431, BxPC3, C5N, Caco-2, Capan-1, CC531, CFPAC, CHO, CHO K1, COS-1, COS-7, CV-1, EAHY, EAHY 926, F98, GH3, H-295 R, H-4-II-E, HACAT, HACAT A131, HEK, HEL, HeLa, Hep G2, High Five, Hs 766T, HT29, HUV-EC R24, HUV-EC-C, IEC 17, IEC 18, Jurkat, K 562, KARPAS-299, L 929, LIN 175, MAt-LYLU, MCF-7, MNEL, MRC-5, MT4, N64, NCTC 2544, NDCK II, Neuro 2A, NIH 3T3, NT2/D1, P19, SF9, SK-UT-1, ST, SW 480, SWU-2 OS, U-373, U-937, and Y-1. Other suitable cells are those known to the one of skill in the art.

The procedure for introducing a transgene into a recipient cell is called transfection. In general a cell line is transfected by any of the means mentioned above, wherein the transgene is operatively linked to a selectable marker. Following transfection cells are grown e.g. for some days in enriched media and then switched to selective media. Transfected cells exhibit resistance to the selection and are able to grow, whereas non-transfected cells die in general. Examples for selectable markers include puromycin, zeocin, neomycin (neo) and hygromycin B, which confer resistance to puromycin, zeocin, aminoglycoside G-418 and hygromycin, respectively. However, other selection methods known to the skilled person may be also suitable.

As described above, cells may be cultured in a suitable medium (e.g. commercially available medium including serum and antibiotics). Cells are usually cultured in a suitable atmosphere (5% CO₂), relative humidity (e.g. 90 %) and temperature (e.g. at 37°C). For high-through-put screening and/or convenient handling cells may be cultivating in multiwell plates such as 96 well plates, 24 well plates etc. The Flp-In-CHO cells in the examples were seeded in complete medium and were cultured for 24 hrs at 37°C and 5% CO₂.

Media with or without FCS may be used for incubating the cells. In the examples, the medium is F-12K nutrient mixture, Pen/Strep, 10% FCS and Hygromycin (300µg/ml).

In a preferred embodiment, the test system in addition comprises means for quantitating cells, in particular viable cells. This allows correlating the number of cells, with the signal mediated the label, in particular the fluorescence, originating from DDR polypeptide detection. Thus, in a preferred embodiment, in addition at least one reagent for staining the cells, in particular for staining cell nuclei is used. In particular, the Hoechst dye HOE 33342 may be used, as shown in the examples. This step can be performed after fixing and permeabilizing the cells. Preferably, this step is performed after adding the binding partner capable of selectively recognizing phospho-tyrosine to the cells. In particular, this step is carried out simultaneously with adding the binding partner capable of selectively recognizing the binding partner selectively recognizing phospho-tyrosine to the cells. In particular, the Hoechst dye HOE 33342 may be used for staining cell nuclei. Therefore, in one embodiment, at least one reagent for staining the cells is added, in particular at least one reagent for staining cell nuclei is added, wherein this step is carried out after step d) of the method of the invention, preferably simultaneously with step f1), f2)i) or f2ii) of the method of the invention.

In another preferred embodiment, the binding partner capable of selectively recognizing phospho-tyrosine is an anti-tyrosine antibody or an antibody fragment. Such antibodies are known in the art.

In another preferred embodiment, the test system comprises another binding partner which is labeled and which is capable of selectively recognizing the binding partner capable of selectively recognizing phospho-tyrosine. Thus, the variant of b2) of the test system and variant f2) of the method of the invention is preferred. Preferably, this another binding partner capable of selectively recognizing the binding partner capable of selectively recognizing phospho-tyrosine is an antibody or antibody fragment. Thus, in this embodiment, this another binding partner is a labeled secondary antibody recognizing the primary anti-phospho-tyrosine antibody.

Therefore, in an especially preferred embodiment, a primary antibody or antibody fragment selectively recognizing phospho-tyrosine, and a secondary labeled antibody or antibody fragment selectively recognizing the primary antibody are used. In the examples, anti-phospho-tyrosine antibody produced in rabbit (Sigma, Cat. No. T-1575) was used as primary antibody. This antibody is a preferred antibody according to the invention. In another preferred embodiment, Alexa Fluor F(ab)2 fragment goat anti-rabbit IgG (Alexa) may be used as labeled secondary antibody. This antibody was shown to be useful according to the invention in the Examples.

In a preferred embodiment, a blocking buffer is added and incubated prior to adding the binding partner selectively recognizing phospho-tyrosine, in particular the anti-phospho-tyrosine antibody or antibody fragment. The blocking buffer may be added after fixing the cells and permeabilizing the cells. Blocking buffers are well known in the art. For example, the blocking buffer described in example 1 may be used.

Herein, the term antibody or antibody fragment is understood as meaning antibodies, antibody fragments, or antigen-binding parts thereof, in particular complementarity-determining regions (CDRs) of antibodies, which have been prepared recombinantly and, where appropriate, modified, such as chimaeric antibodies, humanized antibodies, multifunctional antibodies, bispecific or oligospecific antibodies, single- chain antibodies and Fab, Fab' or F(ab)₂ fragments (see, for example, EP-B1-0 368 684, US 4,816,567, US 4,816,397, WO 88/01649, WO 93/06213 or WO 98/24884), fragments produced by a Fab expression library, anti-idiotypic (anti-ld) antibodies, and epitope-binding fragments of any of the above.

As an alternative to the classical antibodies it is also possible, for example, to use protein scaffolds as binding partners, e.g. anticalins which are based on lipocalin (Beste et al. (1999) Proc. Natl. Acad. Sci. USA, 96, 1898-1903). The natural ligand-binding sites of the lipocalins, for example the retinol-binding protein or the bilin-binding protein, can be altered, for example by means of a "combinatorial protein design" approach, in such a way that they bind to selected haptens (Skerra, 2000, Biochim. Biophys. Acta, 1482, 337-50). Other known protein scaffolds are known as being alternatives to antibodies for molecular recognition (Skerra (2000) J. Mol. Recognit., 13, 167-187).

According to the present invention, at least one labeled binding partner is used. The label may be any covalently or non-covalently bound chemical entity, which can be used for detection. Common labeling methods may be used for labeling of one or more functional groups on the surface of the binding partners. These could be for example the primary amino groups, present at the N-terminal of each polypeptide chain and the side chain of lysine residues; sulphhydryl groups, present on cysteine residues made available by treating disulphide bonds with reducing agent or by modifying lysine residues with a reagent such as SATA. The binding partner may be labeled with a series of different agents, e.g. such as biotin (for avidin-biotin chemistry), enzymes, activated fluorescent dyes for labeling amines, sulphhydryls or other functional groups with e.g. FITC, fluorescein, rhodamine, Cy dyes or Alexa fluors. Radioactive label such as ³H, ³²P, ³⁵S, ¹²⁵I or ¹⁴C as well as common enzyme labels including penicillinase, horseradish peroxidase and alkaline phosphatase may be used as well.

In a preferred embodiment, a fluorescently labeled binding partner, in particular a fluorescently labeled antibody or antibody fragment is used. Preferably, the binding partner, which is a labeled binding partner, and which is capable of selectively recognizing the binding partner recognizing phospho-tyrosine, is a fluorescently labeled binding partner. Thus, in an especially preferred embodiment, a fluorescently labeled antibody or antibody fragment specifically recognizing an anti-phospho-tyrosine antibody or antibody fragment is used. In this embodiment, the anti-phospho-tyrosine antibody does not need to be labeled, preferably, the anti-phospho-tyrosine antibody is not labeled or labeled with a label different from the label of the antibody or antibody fragment specifically recognizing an anti-phospho-tyrosine antibody or antibody fragment.

Fluorescence is an optical phenomenon, wherein the molecular absorption of a photon triggers the emission of another photon with a longer wavelength. The energy difference between the absorbed and emitted photons ends up as molecular vibrations or heat. Usually, the absorbed photon is in the ultraviolet range, and the emitted light is in the visible range, but this depends on the absorbance curve and Stokes shift of the particular fluorophore. The number of fluorescence applications is growing in the biomedical biological and related sciences. Methods of analysis in these fields are also growing, albeit with increasingly unfortunate nomenclature in the form of acronyms such as: FLIM, FLI, FLP, CALI, FLIE, FRET, FRAP, FCS, PFRAP, smFRET, FIONA, FRIPS, SHREK, SHRIMP, TIRF. Most of these techniques rely on fluorescence microscopes. These microscopes use high intensity light sources, usually mercury or xenon lamps, LEDs, or lasers, to excite fluorescence in the samples under observation. Optical filters then separate excitation light from emitted fluorescence, to be detected by eye, or with a (CCD) camera or other light detectors (photomultiplier tubes, spectrographs, etc).

Preferably, a fluorescent dye is used as label in order to detect the effect of a signal in question (here the collagen-induced auto-/transphosphorylation of a DDR polypeptide). The fluorescent dye comprises a fluorophore. A fluorophore, in analogy to a chromophore, is a component of a molecule which causes a molecule to be fluorescent. It is a functional group in a molecule which will absorb energy of a specific wavelength and re-emit energy at a different (but equally specific) wavelength. The amount and wavelength of the emitted energy depend on both the fluorophore and the chemical environment of the fluorophore. Fluorescein isothiocyanate, a reactive derivative of fluorescein, has been one of the most common fluorophores chemically attached to other, non-fluorescent molecules to create new and fluorescent molecules for a variety of applications. Other historically common fluorophores are derivatives of rhodamine, coumarin and cyanine. Examples fluorescent dyes include without limitation 7-amino-actinomycin D, acridine orange, acridine yellow, Alexa Fluor, AnaSpec, auramine O, auramine-rhodamine stain, benzanthrone, 9,10-bis(phenylethynyl)anthracene, 5,12-bis(phenylethynyl)naphthacene, CFDA-SE, CFSE, calcein, carboxyfluorescein, 1-chloro-9,10-bis(phenylethynyl)anthracene, 2-chloro-9,10-bis(phenylethynyl)anthracene, coumarin, cyanine, DAPI, Dioc6, DyLight Fluor, ethidium bromide, fluorescein, Fura-2, Fura-2-acetoxymethyl ester, green fluorescent protein, Hilyte Fluor, Hoechst stain, Indian yellow, Indo-1, luciferin, perylene, phycobilin, phycoerythrin, phycoerythrobilin, propidium iodide, pyranine, rhodamine, RiboGreen, rubrene, ruthenium(II) tris(bathophenanthroline disulfonate), SYBR Green, stilbene, sulforhodamine 101, TSQ, Texas Red, umbelliferone and yellow fluorescent protein. Preferred is the use of Alexa Fluor, as described in the examples.

In the context of the present invention, the fluorescent dye is covalently or non-covalently bound to the binding partner(s) of the present invention, preferably, it is covalently bound.

For the method of the invention any suitable detection may be used. The detection is system is adapted to detect the label. In an embodiment the assay is a DELFIA (dissociation enhanced lanthanide fluoro immuno assay), a FRET (fluorescence resonance energy transfer) assay, or TR-FRET (time-resolved fluorescence resonance energy transfer) assay.

DELFIA (dissociation enhanced lanthanide fluoro immuno assay)-based assays are solid phase assay. The antibody is usually labeled with Europium or another lanthanide and the Europium fluorescence is detected after having washed away un-bound Europium-labeled antibodies.

In a preferred embodiment, the detection means are suitable for detection of fluorescence. Such detection means are used to detect the fluorescent label.

In a preferred embodiment, the means for detection are automated or semi-automated. In particular, the means for detection is a confocal microscope. In particular, the ImageXpress Ultra Confocal High Content Screening System (Molecular Devices) may be used, as described in the Examples. Also, fluorescence microplate cytometry may be used in another embodiment, e.g. Acumen Explorer^{™}.

The solid support for fixing cells at least one solid support for fixing the cells and for culturing the cells is in a preferred embodiment a multiwell plate, in particular comprising 12 wells or more, 24 wells or more, 48 wells or more, 96 wells or more, 384 wells or more.

The solid support is suitable for both culturing the cells thereon, and for fixing the cells thereon, using suitable reagent(s).

The fixing of cells may be performed with suitable reagents known in the art. In particular, paraformaldehyde may be used, as also described in the Examples. Also, an orthovanadate salt, in particular sodium orthovanadate may in addition be added to the fixing reagent in order to block phosphatase acitvity, as also described in the Examples.

For cell permeabilization, suitable reagents known in the art may be used. In particular, Triton-X-100 may be used, as also described in the Examples.

In a preferred embodiment, the test system comprises:
a) at least one mammalian cell, preferably at least one CHO cell, more preferably at least one Flp-In-CHO cell expressing at least one mammalian DDR polypeptide selected from mammalian DDR1a, DDR1b, DDR1c and DDR2 localized in the plasma membrane of the cell,
b) an anti-phospho-tyrosine antibody or antibody fragment
c) a fluorescently labeled antibody or antibody fragment selectively binding to the anti-phospho-tyrosine antibody or antibody fragment,
d) a well plate comprising at least 12 wells,
e) at least one mammalian collagen,
f) optionally at least one compound suspected of altering the collagen-induced auto-/transphosphorylation of the at least one DDR polypeptide or functional derivative thereof, and
g) detection means for quantitative detection of fluorescence in the wells of d).

In another preferred embodiment, the method of the invention is carried out using these components of the test system.

The test systems and methods of the invention make use of at least one collagen. The collagen is employed to mediate the collagen-induced auto-/transphosphorylation of the DDR polypeptides. Collagen is a group of naturally occurring proteins. In nature, it is found exclusively in animals, especially in the flesh and connective tissues of mammals. A distinctive feature of collagen is the regular arrangement of amino acids in each of the three chains of these collagen subunits. The sequence often follows the pattern Gly-Pro-X or Gly-X-Hyp, where X may be any of various other amino acid residues.

In a preferred embodiment, the collagen is collagen of mammalian origin, in particular collagen from human, rat or mouse. Most preferably, the collagen is human collagen. 29 types of collagen were identified, collagen I to XXIX, with collagen I, II, III, IV and V being the most common ones.

In a preferred embodiment, the collagen is selected from collagen type I, collagen type II, collagen type III or collagen type IV, or mixtures thereof. These collagens are especially preferred in case of DDR1 isoforms and orthologs. In a preferred embodiment, the collagen is selected from human collagen type I, human collagen type II, human collagen type III and human collagen type IV or mixtures thereof. In another preferred embodiment, rat collagen type I, rat collagen type II, rat collagen type III or rat collagen type IV, or mixtures thereof may be used. In another preferred embodiment, fibrillar collagens are used. Such collagens are fibril-forming. Such collagens are especially used for DDR2 isoforms and orthologs.

In a preferred embodiment, the at least one collagen to be added according to the method of the invention, and as present in the test systems of the invention, is dissolved in a suitable solvent, preferably in an acidic solvent. Suitable solvents are known in the art, in particular acetic acid and/or HCl, e.g. 0,1 N HCL, may be used as acidic solvents. In particular, acetic acid, more preferably 0,1% acetic acid or 0,1N acetic acid, as described in Example 1, may be used. In a particularly preferred embodiment, the at least one collagen is dissolved in the solvent prior to adding according to the method of the invention.

The duration of incubation with collagen may vary and may be optimized for each incubation medium, DDR polypeptide and collagen. Typically, the collagen incubation takes place for about 5 minutes to about 4 days. In a preferred embodiment, collagen incubation is performed for about 10 minutes to about 60 hours, more preferably for about 1 to about 36 hours, even more preferred for about 1 to about 25 hours, most preferred for about 2 to about 18 hours. Optimized collagen incubation times of about 2 hours and about 18 hours have been determined in the examples for specific experimental setups of the invention.

"About" according to the present invention is understood as meaning the experimental error range, in particular ± 5% or ± 10%.

The incubation of the cells may be carried out in various cellular buffers. A suitable cellular buffer allows the cells to be viable. Therefore, complete medium or medium without FCS may be used according to the invention. In a preferred embodiment, the cellular buffer is medium, which optionally comprises FCS. FCS may be present in various concentrations. In particular, the medium may contain about 0,1% to 20% FCS, in particular about 1% to about 10% FCS, as exemplified in Example 1. FCS is understood to mean fetal calf serum.

In a preferred embodiment, the method is carried out as a mid-throughput assay or a high-throughput assay. Preferably, the method is carried out as a high-throughput assay. In this method, a large number of compounds are screened in whole-cell assays. Typically, these screenings are carried out in at least 96 well plates using automated, robotic station based technologies or in higher-density array ("chip") formats.

The method of the invention may be used for screening for a diagnostic and/or medicament for diagnosing, preventing and/or treating a disease involving DDR polypeptide(s), respectively, particularly for preventing and/or treating any of the diseases described above. If used for screening purposes the compound tested may be a compound, the function of which is still unknown or not yet related to the DDR polypeptide(s). Also, the method may be used for the identification of new compounds altering collagen-induced auto-/transphosphorylation. Alternatively, compounds known to alter collagen-induced auto-/transphosphorylation may be tested for their efficiency using the method of the invention, in particular for quantification.

In a preferred embodiment, a quantitative detection is performed. In particular, the detection means may be equipped with software to transform experimental parameters in quantitative values.

In a particularly preferred embodiment, the concentration-dependent alteration of collagen-induced auto-/transphosphorylation of the at least one DDR polypeptide or functional derivative thereof is determined. Therefore, the alteration at least two different concentrations of the compound is preferably determined.

The alteration may be an inhibition or activation, preferably an inhibition. In particular, % phosphorylation of the DDR polypeptide(s) is determined. In particular, IC₅₀ values in case of inhibitory compounds are determined.

As described above, the compound suspected of altering the collagen-induced auto-/transphosphorylation of a DDR polypeptide or functional derivative thereof may be a substance of any chemical nature. It may already be known as a drug or medicament or diagnostic for a disease. Alternatively, it may be a known chemical compound not yet known to have a therapeutic or diagnostic effect in another embodiment and the compound may be a novel or so far unknown chemical compound. The agent may be also a mixture of substances or compounds.

In one embodiment, the compound is provided in form of a chemical compound library. Chemical compound libraries include a plurality of chemical compounds and have been assembled from any of multiple sources, including chemical synthesized molecules or natural products, or have been generated by combinatorial chemistry techniques. They are especially suitable for high-throughput screening and may be comprised of chemical compounds of a particular structure or compounds of a particular organism such as a plant. In the context of the present invention, the chemical compound library is preferably a library comprising proteins and polypeptides or small organic molecules. Preferably a small organic molecule is less than 500 daltons in size, particularly a soluble, non-oligomeric, organic compound.

In another preferred embodiment, the compound is a biological molecule, like an antibody, antibody fragment, antibody mimetic, peptide, or RNA molecule or libraries thereof.

The compound altering the collagen-induced auto-/transphosphorylation of a DDR polypeptide or functional derivative thereof may be an inhibitor or an activator. Preferably, it is an inhibitor.

In a preferred embodiment, the inhibitor inhibits collagen-induced auto-/transphosphorylation of a DDR polypeptide or functional derivative by at least 5 %, more preferably 10 %, 30 %, 50 %, 70 % or 90 %, of the control. The collagen-induced auto-/transphosphorylation can be determined as shown in Example 1.

In another preferred embodiment, the inhibitor has an IC₅₀ value of less than about 1µg/µl, preferably less than about 100ng/µl, more preferably less than about 10ng/µl, most preferably less than 2 ng/µl. The IC₅₀ value can be determined as shown in Example 1.

In another preferred embodiment, the inhibitor has an IC₅₀ value of less than about 10 µM, preferably less than about 1 µM, more preferably less than about 0,1 µM, even more preferably less than about 10 nM most preferably less than about 7 nM.

The compound may be added at any time point before fixing the cells of on the solid support and permeabilizing the fixed cells, and optionally washing the cells. In a preferred embodiment, the compound is added before, simultaneously or after the addition of collagen. The experiments below show, that the test systems and methods allow the determination of IC₅₀ values of the collagen-induced auto-/transphosphorylation of DDR1 in a cellular system by use of a suitable medium without FCS or with FCS, in various concentrations. Also, any DDR1 or DDR2 orthologs or isoforms can be used including isoforms and orthologs from mammalian or non-mammalian organisms. In addition, various collagens may be used insofar as these activate the auto/transphosphorylation of DDR. Also, various incubation times for the administered collagens can be used and, also, the incubation conditions like, for example, pre-incubation of the inhibitory or activating reagent or simultaneous administration of the modulating compound with the collagen can be performed. Also, the test systems and methods of the present invention can be used for quantitative determination of chemical reagents or biological molecules like, for example, peptides or antibodies which modulate the collagen-induced auto/transphosphorylation of a DDR polypeptide. Moreover, the test systems and methods allow performing the methods in various assay formats like, for example, a 12-well, 24-well, 48-well, 96-well, or 384-well plate format. The assay can be performed in a middle- or a high-throughput assay format.
SEQ ID No. 1 shows the cDNA encoding human DDR1a (NM_001954; http://www.ncbi.nlm.nih.gov/entrez/viewer.fcgi?val=NM_001954)
SEQ ID No. 2 shows the human DDR1a polypeptide sequence (NM_001954; http://www.ncbi.nlm.nih.gov/entrez/viewer.fcgi?val=NM_001954)
SEQ ID No. 3 shows the cDNA encoding human DDR1b (NM_013993; http://www.ncbi.nlm.nih.gov/entrez/viewer.fcgi?val=NM_013993) DDR1 b (913AA): NM_013993
SEQ ID No. 4 shows the human DDR1 b polypeptide sequence (NM_013993; http://www.ncbi.nlm.nih.gov/entrez/viewer.fcgi?val=NM_013993)
SEQ ID No 5 shows the cDNA encoding human DDR1c (NM_013994; http://www.ncbi.nim.nih.gov/entrez/viewer.fcgi?val=NM_013994)
SEQ ID No. 6 shows the human DDR1c polypeptide sequence (NM 013994; http://www.ncbi.nlm.nih.gov/entrez/viewer.fcgi?val=NM_013994)
SEQ ID No. 7 shows a cDNA encoding human DDR2 (NM_001014796.1 (http://www.ncbi.nlm.nih.gov/nuccore/NM_001014796.1)
SEQ ID No. 8 shows the human DDR2 polypeptide sequence (NM_001014796.1 (http://www.ncbi.nlm.nih.gov/nuccore/NM_001014796.1)
SEQ ID No. 9 shows a cDNA encoding a murine DDR1 polypeptide (transcript variant 2; NM_172962; http://www.ncbi.nlm.nih.gov/nuccore/NM_172962)
SEQ ID No. 10 shows a murine DDR1 polypeptide sequence (NM_172962; http://www.ncbi.nlm.nih.gov/nuccore/NM_172962)

### Description of Figures

Figure 1A shows collagen-induced auto-/transphosphorylation of hDDR1a as determined in + and- FCS CHO medium.
Figure 1 B shows collagen-induced-auto-/trans-phosphorylation of hDDR1c as determined in -FCS CHO medium.
Figure 2 shows the concentration-dependent inhibition of auto-/transphosphorylation of human DDR1a in CHO medium. Rat collagen I-induced hDDR1a phosphorylation is inhibited in a dose-dependent manner by a specific commercially available anti-DDR1 antibody. IC₅₀ was determined as about 0,5 ng/µl.
Figure 3A shows that clinical inhibitors of abl kinase or the kinases of the src family significantly inhibit the collagen-induced auto-/transphosphorylation of human DDR1. For Dasatinib as inhibitor, IC₅₀ was determined as about 10 nM.
Figure 3B shows that rat collagen I-induced mDDR1a phosphorylation is inhibited in a dose-dependent manner by a specific anti-DDR1 pAB antibody. IC₅₀ was determined as about 2,4 ng/µl.
Figures 4A ,4B and 4C show that human collagen I induces hDDR1a tyrosine phosphorylation and that human collagen I-induced hDDR1a phosphorylation is inhibited as well in a dose-dependent manner by a specific anti-DDR1 antibody as compared to induction by rat collagen type I (rat tail) .
Figures 5A and 5B show that imatinib (Figure 7A) and nilotinib (Figure 7B) inhibit rat collagen-induced hDDR1 phosphorylation. IC₅₀ of imatinib is about 0.89 µM (10% FCS); IC₅₀ of nilotinib is about 0,096 µM (10% FCS).
Figure 6 shows a typical experimental protocol followed in the Examples.

### Examples

### Example 1

For the setup of the assay system, Flp-In CHO cells were stably transfected with the human full-length gene of the isoform DDR1a according to SEQ ID No. 1. These cell lines express the corresponding DDR1 a isoform in the correct cellular localization in the plasma membrane. The expression was confirmed by Western Blot analysis and the correct physiological localization was detected by immunofluorescence experiments. In the parental cell line, no DDR1 expression could be detected using the above methods. The cells were seeded in a black 96-well or 384-well microtiter plate with clear bottom in complete medium which contains 1-10% FCS. After incubation of the cells over night (16-24 hours) in a CO2 (5%) incubator at 37 °C, the cells were treated with either collagen type I, which is commercially available for 2 hours. If necessary, the compound, which is suspected to alter the activity of DDR1, can be administered in various doses for the detection of the corresponding IC₅₀ value before or at the same time as administering collagen. After that, the cells were fixed and permeabilized. Then, they were incubated in blocking buffer, and were subsequently incubated over night with an anti-phospho-tyrosine antibody. This antibody binds to all phosphorylated tyrosines present in the cells. On the following day, a corresponding secondary antibody, which is labeled with a fluorescent dye, was administered to the cells and incubated for one hour. At the same time, the cell nuclei were treated with a dye which is specific for cell nuclei.

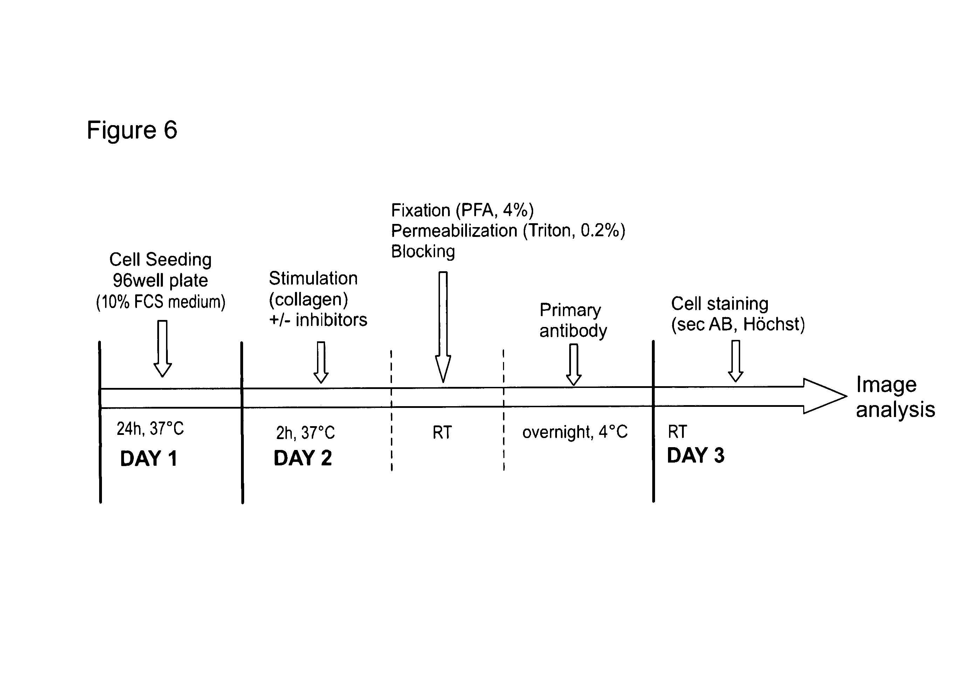

The intensity of the so-labeled cells was detected with a corresponding measuring system like, for example, ImageXpress of Molecular Devices or the system Acumen. By analysis of the measured data with a specified software protocol, the percentage of DDR1 phosphorylation was determined. A schematic overview of the experiments is shown in Figure 6.

Using this approach, IC₅₀ values relating to the inhibition of phosphorylation of DDR1 polypeptides of compounds like antibodies or small chemical molecules was determined.

Following reagents were used in the experiments:
Vector: pcDNA5-FRT-TO_DEST (Invitrogen Life Technologies)
Cells: CHO Flp-In wt for generating stably expressing CHO-hDDR1a, CHO-mDDR1a, CHO-hDDR1b, and CHO-hDDR1c.
Media: F-12K Nutrient mixture (Kaighn's Modification; Gibco, Cat. No.:21127), Pen/Strep (PAN), 10% FCS (PAN), Hygromycin 300µg/ml (Roche Diagnostics Deutschland GmbH) Buffer: PBS
Reagents: Collagen I (rat tail; Sigma); Collagen human VitroCol (AdvancedBiomatrix); Acetic acid 0,1%; Sodium orthovanadate 20µM; paraformaldehyde 4%; Triton X-1 00; Tween 20; Odyssey Blocking Buffer (LI-COR, Cat. No.: P2151); Anti-Phospho-Tyrosine rabbit (Sigma, Cat. No.: T-1575); Anti-Phospho-Tyrosine mouse 4G10 (Upstate, Cat. No.: 05-321); Alexa Fluor 488 F(ab)2 fragment goat anti-rabbit IgG (Invitrogen, Cat. No.: A11070); Alexa Fluor 488 F/ab)2 fragment goat anti-mouselgG (Invitrogen Cat. No.: A11017); HOE 33342
(Invitrogen, Cat. No.: H3570)
Cell culture plates: BD Falcon, 96 well
Reference compounds: Anti-human DDR1 Antibody (R&D Systems, Cat No.: AF2396) Normal Goat IgG (R&D Systems, Cat No.: AB-108-C)
Dasatinib (Sprycel, BMS-354825)

### Example 2

The corresponding experiments were performed with human DDR1 b according to SEQ ID No. 3 and DDR1c according to SEQ ID No. 5 accordingly.

### Example 3

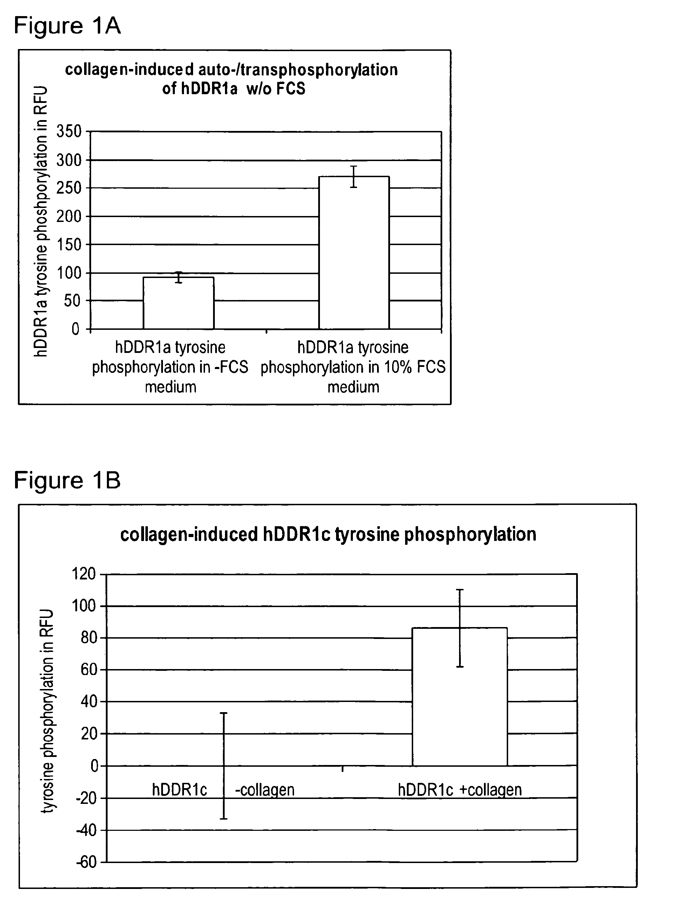

The experimental setup was shown to be applicable to different cellular buffer conditions: The quantitative determination of the auto/transphosphorylation of DDR1 in a cell-based system by use of a CHO-Flp-in cell line which stably expresses the full-length DDR1 protein was performed using the CHO medium with different FCS concentrations for incubation during collagen-induced auto-/transphosphorylation of DDR1. The result for these different cellular buffers is shown in Figure 1. It could be shown, that the method of the invention can be performed using CHO medium with different FCS concentrations as cellular buffer.

### Example 4

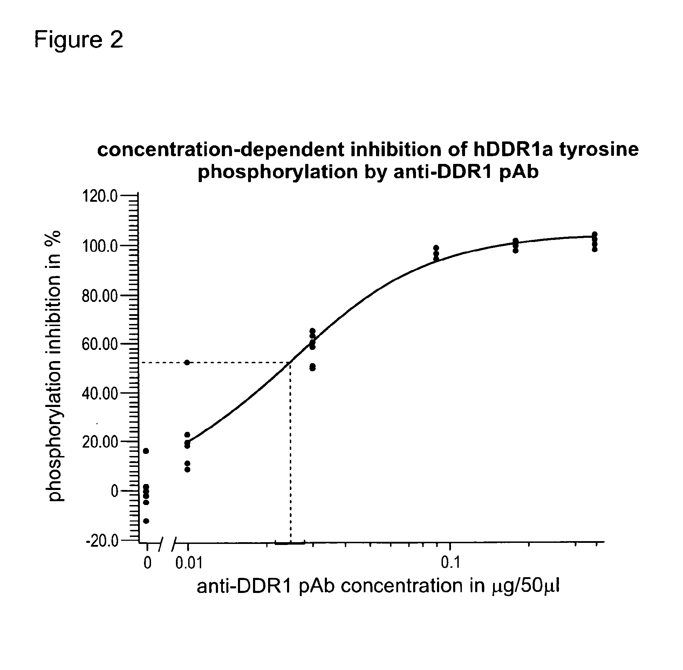

The effect of potential modulators of collagen-induced DDR1 and DDR2 activity can be determined using the assay. This can be performed in a medium- to high-throughput assay format. In the experiment, a commercially available antibody anti-hDDR1 (R&D Systems, Cat No. AF2396; Polyclonal Goat IgG; immunogen: Mouse myeloma cell line NS0derived recombinant human DDR1, Asp21-Thr416), which is directed against the extracellular domain of DDR1, was shown to inhibit the collagen-induced phosphorylation of DDR1 under various cellular buffer conditions, dependent on the concentration of the modulator. Various concentrations of the antibody were added. It could be determined that the IC₅₀ value in CHO medium is about 0,5 ng/µl. The result is shown in Figure 2.

### Example 5

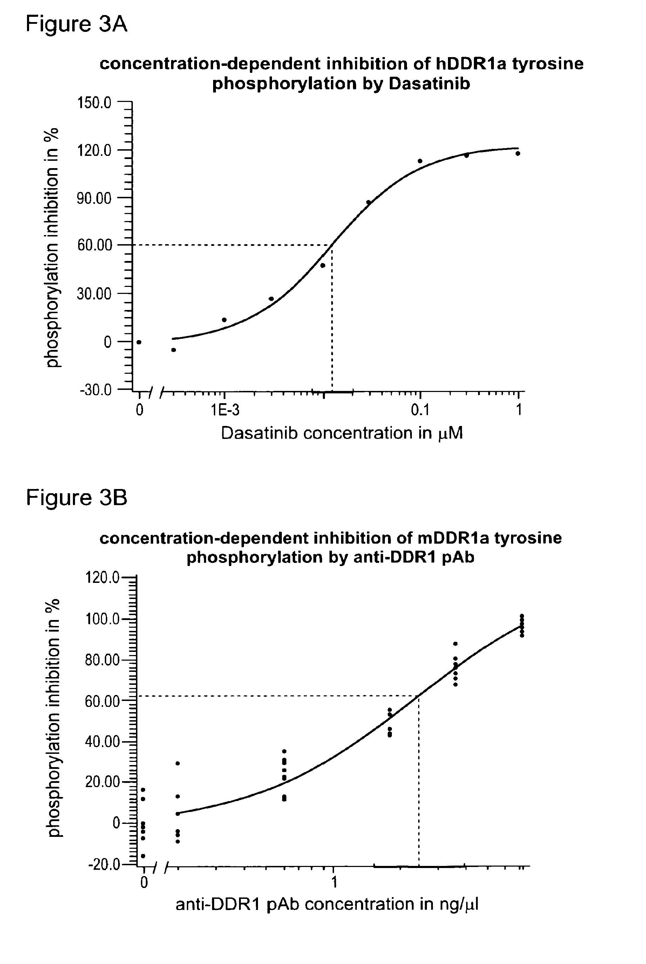

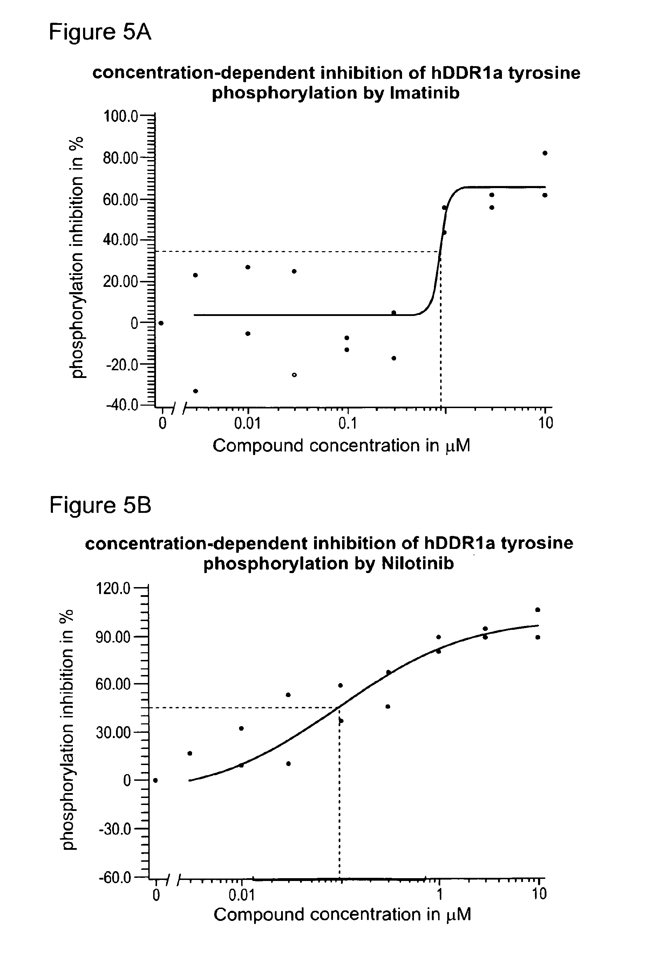

Clinical inhibitors of the ABL kinase or the kinases of the SRC family were shown to significantly inhibit the collagen-induced phosphorylation of DDR1. Dasatinib, which is an inhibitor of this class, was determined to have an IC₅₀ of about 7 nM (10% FCS; see Figure 3A). Figures 5A and 5B show that imatinib (Figure 5A) and nilotinib (Figure 5B) inhibited rat collagen-induced hDDR1 phosphorylation. The IC₅₀ of imatinib is about 0,89 µM (10% FCS); the IC₅₀ of nilotinib was determined to be about 0,096 µM (10% FCS).

### Example 6

In this experiment, Flp-In-CHO cells stably overexpressing mouse full-length DDR1 a were used. The cells were stimulated with rat collagen for 2 hours. As an inhibitor, a specific anti-DDR1 pAB antibody was used. The result is shown in Figure 3B. The IC₅₀ was calculated to be about 2,4 ng/µl.

### Example 7

In this experiment, Flp-In-CHO cells stably overexpressing human full-length DDR1a according to SEQ ID No. 1 were used. The cells were stimulated with rat collagen for 2 hours. As an inhibitor, a specific commercially available anti-DDR1 antibody as described in Example 1 was used. The result is shown in Figure 2. The IC₅₀ for the antibody was calculated to be about 0,025 µg/well, corresponding to about 0,5ng/µl.

### Example 8

For the first part of the experiment, human collagen type I (neonatal fibroblasts) was used for stimulating human DDR1 phosphorylation. The experiment was performed in normal medium. No induction of phosphorylation was observed at 2 or 6 hours. Optimal collagen incubation time for phosphorylation was determined to be about 18 hours.

For the second part of the experiment, rat collagen type I (rat tail) was used for stimulating human DDR1 phosphorylation. Optimal collagen incubation time for phosphorylation was determined to be about 2 hours.

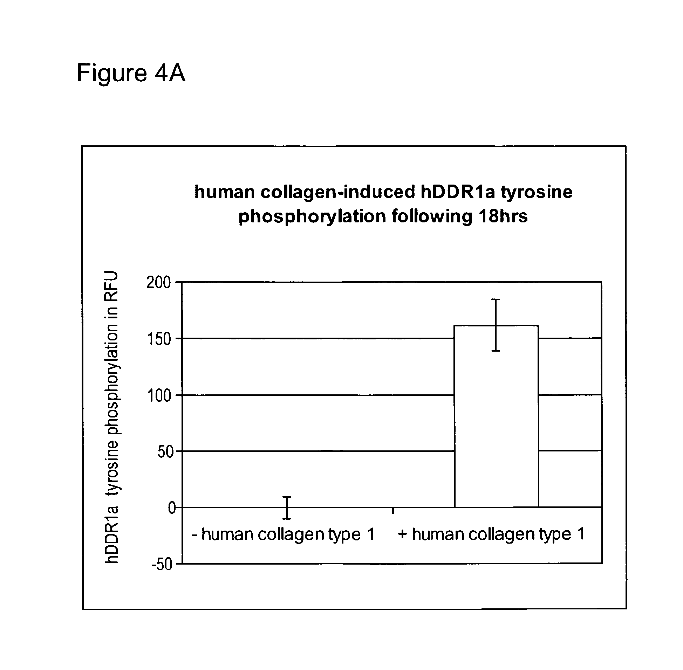

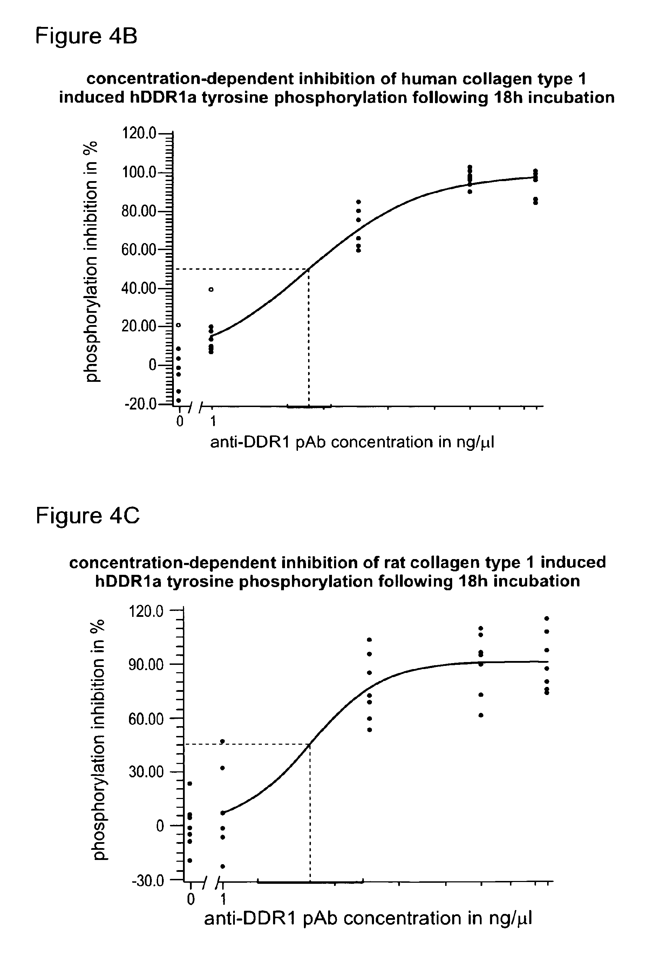

For both experimental parts, the IC₅₀ for the specific anti-DDR1 antibody pAB was determined. For the experiment with human collagen, the IC₅₀ was about 1,8 ng/µl, for the experiment with rat collagen, the IC₅₀ was about 1,7 ng/µl. Figures 4A and 4B show that human collagen I-induced DDR1 phosphorylation is inhibited as well in a dose-dependent manner by a specific anti-DDR1 antibody as compared to induction by rat collagen type I (rat tail).

## Claims

1. A test system for determining the Discoidin domain receptor (DDR) collagen-induced auto-/transphosphorylation activity comprising:
a) at least one cell containing at least one DDR polypeptide or functional derivative thereof localized in the plasma membrane of the cell,
b) either b1) or b2):
b1) a binding partner capable of selectively recognizing phospho-tyrosine,
which binding partner is a labeled binding partner,
b2) a binding partner capable of selectively recognizing phospho-tyrosine
and another binding partner, which is a labeled binding partner, and which is capable of selectively recognizing the binding partner recognizing phospho-tyrosine,
c) at least one reagent for fixing the cells on a solid support and permeabilizing the cells
d) at least one solid support for fixing the cells and for culturing the cells,
e) at least one collagen,
f) optionally at least one compound suspected of altering the collagen-induced auto-/transphosphorylation of the at least one DDR polypeptide or functional derivative thereof, and
g) means for detecting the binding partner of b) or c).

2. The test system according to claim 1,
a) wherein the cell and/or the DDR polypeptide are of mammalian origin, and/or
b) wherein at least one DDR polypeptide or functional derivative thereof are expressed in a mammalian cell, in particular a CHO cell, and/or
c) wherein the DDR polypeptide is selected from DDR1a, DDR1b, DDR1c and DDR2.

3. The test system according to any of the preceding claims, wherein the DDR polypeptide is a human polypeptide and/or selected from the polypeptides according to SEQ ID No. 2, SEQ ID No. 4, SEQ ID No. 6, SEQ ID No. 8, or SEQ ID No. 10, or combinations thereof.

4. The test system according to any of the preceding claims, wherein the binding partner selectively recognizing phospho-tyrosine of claim 1 b1) or 1 b2) is an anti-tyrosine antibody or antibody fragment.

5. The test system according to any of the preceding claims, wherein the labeled binding partner is a fluorescently labeled binding partner.

6. The test system according to any of the preceding claims, wherein the detection means of claim 1g) are suitable for detection of fluorescence.

7. The test system according to any of the preceding claims, wherein the collagen is selected from collagen type I, collagen type II, collagen type III or collagen type IV, or mixtures thereof.

8. The test system according to any of the preceding claims, wherein the cells are recombinant cells, and/or wherein the parental cells do not contain DDR polypeptides localized in the plasma membrane of the cell.

9. The test system according to any of the preceding claims, wherein the test system in addition comprises at least one reagent for staining the cells, in particular for staining cell nuclei.

10. The test system according to any of claims 1 to 9 comprising:
a) at least one a mammalian cell, in particular CHO cell, expressing at least one mammalian DDR polypeptide selected from mammalian DDR1 a, DDR1 b, DDR1c and DDR2,
b) an anti-phospho-tyrosine antibody or antibody fragment,
c) a fluorescently labeled antibody or antibody fragment selectively binding to the anti-phospho-tyrosine antibody or antibody fragment,
d) a well plate comprising at least 12 wells,
e) at least one mammalian collagen,
f) optionally at least one compound suspected of altering the collagen-induced auto-/transphosphorylation of the at least one DDR polypeptide or functional derivative thereof,
g) detection means for quantitative detection of fluorescence in the wells of d).

11. A method for identifying a compound altering the DDR collagen-induced auto-/ transphosphorylation activity, particular DDR1a, DDR1b, DDR1c and/or DDR2 collagen-induced auto-/transphosphorylation activity, in particular collagen-induced auto-/transphosphorylation activity of one or more polypeptides having a sequence selected from SEQ ID No. 2, SEQ ID No. 4, SEQ ID No. 6, SEQ ID No. 8, or SEQ ID No. 10, comprising the steps of:
a) culturing at least one cell on a solid support,
which cell contains at least one DDR polypeptide or functional derivative thereof localized in the plasma membrane of the cell,
b) adding to the cell culture of step a) at least one collagen,
in particular wherein the collagen is selected from collagen type I, collagen type II, collagen type III or collagen type IV, or mixtures thereof,
c) incubating the cells of step b) in a cellular buffer,
d) fixing the cells of step c) on the solid support and permeabilizing the fixed cells, and optionally washing the cells,
e) adding at least one compound suspected of altering the collagen-induced auto-/transphosphorylation of the at least one DDR polypeptide or functional derivative thereof before step d),
f) carrying out step f1) or f2):
f1) adding a binding partner to the cells of step d),
which binding partner is capable of selectively recognizing phospho-tyrosine, and
which binding partner is a labeled binding partner,
f2)
i) adding a binding partner to the cells of step d),
which binding partner is capable of selectively recognizing phospho-tyrosine,
ii) adding another binding partner to cells of step i),
which another binding partner is a labeled binding partner, and which another binding partner is capable of selectively recognizing the binding partner capable of selectively recognizing phospho-tyrosine,
g) detecting the labeled binding partner.

12. The method according to claim 11, wherein the cells are mammalian cells transfected with a polynucleotide encoding at least one DDR polypeptide or functional derivative thereof, in particular the cells are transfected CHO cells.

13. The method according to claim 11 or 12, wherein the method is carried out as a high-throughput assay.

14. The method according to any of claims 11 to 13, wherein
i) the labeled binding partner is a fluorescently labeled binding partner,
and/or
ii) wherein quantitative detection is performed, and/or
iii) wherein at least one reagent for staining the cells is added, in particular
wherein at least one reagent for staining cell nuclei is added,
wherein step iii) is carried out after step d) of claim 11, preferably simultaneously with step f1), f2i) or f2ii) of claim 11.

15. The method according to any of claims 11 to 14, wherein
a) collagen incubation is performed for about 10 minutes to about 60 hours, more preferably for about 1 to about 36 hours, even more preferred for about 1 to about 25 hours, most preferred for about 2 to about 18 hours, and/or
b) wherein the cellular buffer for step c) in claim 11 is medium, which optionally comprises FCS.

16. The method according to any of claims 11 to 14,
a) wherein a blocking buffer is added before step f1) or f2i) of claim 11, and/or
b) wherein washing of the cells is carried out after step a), step c), step d), step f1), f2ii) and/or step f2ii) of claim 11.


SEQUENCE LISTING

<110>  Sanofi-Aventis

<120>  Test Systems and methods for identifying a compound altering DDR
       activity

<130>  DE2011/081

<160>  8

<170>  PatentIn version 3.5

<210>  1
<211>  3840
<212>  DNA
<213>  Homo sapiens

<400>  1
gtcttcccct cgtgggccct gagcgggact gcagccagcc ccctggggcg ccagctttgg      60

aggcccccga cagctgctct cgggagccgc ctcccgacac ccgagccccg ccggcgcctc     120

ccgctcccgg ctcccggctc ctggctccct ccgcctcccc cgcccctcgc cccgccgccg     180

aagaggcccc gctcccgggt cggacgcctg ggtctgccgg gaagagcgat gagaggtgtc     240

tgaaggtggc tattcactga gcgatggggt tggacttgaa ggaatgccaa gagatgctgc     300

ccccaccccc ttaggcccga gggatcagga gctatgggac cagaggccct gtcatcttta     360

ctgctgctgc tcttggtggc aagtggagat gctgacatga agggacattt tgatcctgcc     420

aagtgccgct atgccctggg catgcaggac cggaccatcc cagacagtga catctctgct     480

tccagctcct ggtcagattc cactgccgcc cgccacagca ggttggagag cagtgacggg     540

gatggggcct ggtgccccgc agggtcggtg tttcccaagg aggaggagta cttgcaggtg     600

gatctacaac gactgcacct ggtggctctg gtgggcaccc agggacggca tgccgggggc     660

ctgggcaagg agttctcccg gagctaccgg ctgcgttact cccgggatgg tcgccgctgg     720

atgggctgga aggaccgctg gggtcaggag gtgatctcag gcaatgagga ccctgaggga     780

gtggtgctga aggaccttgg gccccccatg gttgcccgac tggttcgctt ctacccccgg     840

gctgaccggg tcatgagcgt ctgtctgcgg gtagagctct atggctgcct ctggagggat     900

ggactcctgt cttacaccgc ccctgtgggg cagacaatgt atttatctga ggccgtgtac     960

ctcaacgact ccacctatga cggacatacc gtgggcggac tgcagtatgg gggtctgggc    1020

cagctggcag atggtgtggt ggggctggat gactttagga agagtcagga gctgcgggtc    1080

tggccaggct atgactatgt gggatggagc aaccacagct tctccagtgg ctatgtggag    1140

atggagtttg agtttgaccg gctgagggcc ttccaggcta tgcaggtcca ctgtaacaac    1200

atgcacacgc tgggagcccg tctgcctggc ggggtggaat gtcgcttccg gcgtggccct    1260

gccatggcct gggaggggga gcccatgcgc cacaacctag ggggcaacct gggggacccc    1320

agagcccggg ctgtctcagt gccccttggc ggccgtgtgg ctcgctttct gcagtgccgc    1380

ttcctctttg cggggccctg gttactcttc agcgaaatct ccttcatctc tgatgtggtg    1440

aacaattcct ctccggcact gggaggcacc ttcccgccag ccccctggtg gccgcctggc 1500
ccacctccca ccaacttcag cagcttggag ctggagccca gaggccagca gcccgtggcc 1560
aaggccgagg ggagcccgac cgccatcctc atcggctgcc tggtggccat catcctgctc 1620
ctgctgctca tcattgccct catgctctgg cggctgcact ggcgcaggct cctcagcaag 1680
gctgaacgga gggtgttgga agaggagctg acggttcacc tctctgtccc tggggacact 1740
atcctcatca acaaccgccc aggtcctaga gagccacccc cgtaccagga gccccggcct 1800
cgtgggaatc cgccccactc cgctccctgt gtccccaatg gctctgccta cagtggggac 1860
tatatggagc ctgagaagcc aggcgccccg cttctgcccc cacctcccca gaacagcgtc 1920
ccccattatg ccgaggctga cattgttacc ctgcagggcg tcaccggggg caacacctat 1980
gctgtgcctg cactgccccc aggggcagtc ggggatgggc cccccagagt ggatttccct 2040
cgatctcgac tccgcttcaa ggagaagctt ggcgagggcc agtttgggga ggtgcacctg 2100
tgtgaggtcg acagccctca agatctggtt agtcttgatt tcccccttaa tgtgcgtaag 2160
ggacaccctt tgctggtagc tgtcaagatc ttacggccag atgccaccaa gaatgccagg 2220
aatgatttcc tgaaagaggt gaagatcatg tcgaggctca aggacccaaa catcattcgg 2280
ctgctgggcg tgtgtgtgca ggacgacccc ctctgcatga ttactgacta catggagaac 2340
ggcgacctca accagttcct cagtgcccac cagctggagg acaaggcagc cgagggggcc 2400
cctggggacg ggcaggctgc gcaggggccc accatcagct acccaatgct gctgcatgtg 2460
gcagcccaga tcgcctccgg catgcgctat ctggccacac tcaactttgt acatcgggac 2520
ctggccacgc ggaactgcct agttggggaa aatttcacca tcaaaatcgc agactttggc 2580
atgagccgga acctctatgc tggggactat taccgtgtgc agggccgggc agtgctgccc 2640
atccgctgga tggcctggga gtgcatcctc atggggaagt tcacgactgc gagtgacgtg 2700
tgggcctttg gtgtgaccct gtgggaggtg ctgatgctct gtagggccca gccctttggg 2760
cagctcaccg acgagcaggt catcgagaac gcgggggagt tcttccggga ccagggccgg 2820
caggtgtacc tgtcccggcc gcctgcctgc ccgcagggcc tatatgagct gatgcttcgg 2880
tgctggagcc gggagtctga gcagcgacca cccttttccc agctgcatcg gttcctggca 2940
gaggatgcac tcaacacggt gtgaatcaca catccagctg cccctccctc agggagcgat 3000
ccaggggaag ccagtgacac taaaacaaga ggacacaatg gcacctctgc ccttcccctc 3060
ccgacagccc atcacctcta atagaggcag tgagactgca ggtgggctgg gcccacccag 3120
ggagctgatg ccccttctcc ccttcctgga cacactctca tgtccccttc ctgttcttcc 3180
ttcctagaag cccctgtcgc ccacccagct ggtcctgtgg atgggatcct ctccaccctc 3240
ctctagccat cccttgggga agggtgggga gaaatatagg atagacactg gacatggccc 3300
attggagcac ctgggcccca ctggacaaca ctgattcctg gagaggtggc tgcgccccca 3360
gcttctctct ccctgtcaca cactggaccc cactggctga gaatctgggg gtgaggagga 3420
caagaaggag aggaaaatgt ttccttgtgc ctgctcctgt acttgtcctc agcttgggct 3480
tcttcctcct ccatcacctg aaacactgga cctgggggta gccccgcccc agccctcagt 3540

cacccccact tcccacttgc agtcttgtag ctagaacttc tctaagccta tacgtttctg 3600

tggagtaaat attgggattg gggggaaaga gggagcaacg gcccatagcc ttggggttgg 3660

acatctctag tgtagctgcc acattgattt ttctataatc acttggggtt tgtacatttt 3720

tggggggaga gacacagatt tttacactaa tatatggacc tagcttgagg caattttaat 3780

cccctgcact aggcaggtaa taataaaggt tgagttttcc acaaaaaaaa aaaaaaaaaa 3840

<210> 2
<211> 876
<212> PRT
<213> Homo sapiens

<400> 2
   Met Gly Pro Glu Ala Leu Ser Ser Leu Leu Leu Leu Leu Leu Val Ala
   1 5 10 15
   Ser Gly Asp Ala Asp Met Lys Gly His Phe Asp Pro Ala Lys Cys Arg
   20 25 30
   Tyr Ala Leu Gly Met Gln Asp Arg Thr Ile Pro Asp Ser Asp Ile Ser
   35 40 45
   Ala Ser Ser Ser Trp Ser Asp Ser Thr Ala Ala Arg His Ser Arg Leu
   50 55 60
   Glu Ser Ser Asp Gly Asp Gly Ala Trp Cys Pro Ala Gly Ser Val Phe
   65 70 75 80
   Pro Lys Glu Glu Glu Tyr Leu Gln Val Asp Leu Gln Arg Leu His Leu
   85 90 95
   Val Ala Leu Val Gly Thr Gln Gly Arg His Ala Gly Gly Leu Gly Lys
   100 105 110
   Glu Phe Ser Arg Ser Tyr Arg Leu Arg Tyr Ser Arg Asp Gly Arg Arg
   115 120 125
   Trp Met Gly Trp Lys Asp Arg Trp Gly Gln Glu Val Ile Ser Gly Asn
   130 135 140
   Glu Asp Pro Glu Gly Val Val Leu Lys Asp Leu Gly Pro Pro Met Val
   145 150 155 160
   Ala Arg Leu Val Arg Phe Tyr Pro Arg Ala Asp Arg Val Met Ser Val
   165 170 175
   Cys Leu Arg Val Glu Leu Tyr Gly Cys Leu Trp Arg Asp Gly Leu Leu
   180 185 190
   Ser Tyr Thr Ala Pro Val Gly Gln Thr Met Tyr Leu Ser Glu Ala Val
   195 200 205
   Tyr Leu Asn Asp Ser Thr Tyr Asp Gly His Thr Val Gly Gly Leu Gln
       210                 215                 220
   Tyr Gly Gly Leu Gly Gln Leu Ala Asp Gly Val Val Gly Leu Asp Asp
   225                 230                 235                 240
   Phe Arg Lys Ser Gln Glu Leu Arg Val Trp Pro Gly Tyr Asp Tyr Val
                   245                 250                 255
   Gly Trp Ser Asn His Ser Phe Ser Ser Gly Tyr Val Glu Met Glu Phe
               260                 265                 270
   Glu Phe Asp Arg Leu Arg Ala Phe Gln Ala Met Gln Val His Cys Asn
           275                 280                 285
   Asn Met His Thr Leu Gly Ala Arg Leu Pro Gly Gly Val Glu Cys Arg
       290                 295                 300
   Phe Arg Arg Gly Pro Ala Met Ala Trp Glu Gly Glu Pro Met Arg His
   305                 310                 315                 320
   Asn Leu Gly Gly Asn Leu Gly Asp Pro Arg Ala Arg Ala Val Ser Val
                   325                 330                 335
   Pro Leu Gly Gly Arg Val Ala Arg Phe Leu Gln Cys Arg Phe Leu Phe
               340                 345                 350
   Ala Gly Pro Trp Leu Leu Phe Ser Glu Ile Ser Phe Ile Ser Asp Val
           355                 360                 365
   Val Asn Asn Ser Ser Pro Ala Leu Gly Gly Thr Phe Pro Pro Ala Pro
       370                 375                 380
   Trp Trp Pro Pro Gly Pro Pro Pro Thr Asn Phe Ser Ser Leu Glu Leu
   385                 390                 395                 400
   Glu Pro Arg Gly Gln Gln Pro Val Ala Lys Ala Glu Gly Ser Pro Thr
                   405                 410                 415
   Ala Ile Leu Ile Gly Cys Leu Val Ala Ile Ile Leu Leu Leu Leu Leu
               420                 425                 430
   Ile Ile Ala Leu Met Leu Trp Arg Leu His Trp Arg Arg Leu Leu Ser
           435                 440                 445
   Lys Ala Glu Arg Arg Val Leu Glu Glu Glu Leu Thr Val His Leu Ser
       450                 455                 460
   Val Pro Gly Asp Thr Ile Leu Ile Asn Asn Arg Pro Gly Pro Arg Glu
   465                 470                 475                 480
   Pro Pro Pro Tyr Gln Glu Pro Arg Pro Arg Gly Asn Pro Pro His Ser
                   485                 490                 495
   Ala Pro Cys Val Pro Asn Gly Ser Ala Tyr Ser Gly Asp Tyr Met Glu
               500                 505                 510
   Pro Glu Lys Pro Gly Ala Pro Leu Leu Pro Pro Pro Pro Gln Asn Ser
           515                 520                 525
   Val Pro His Tyr Ala Glu Ala Asp Ile Val Thr Leu Gln Gly Val Thr
       530                 535                 540
   Gly Gly Asn Thr Tyr Ala Val Pro Ala Leu Pro Pro Gly Ala Val Gly
   545                 550                 555                 560
   Asp Gly Pro Pro Arg Val Asp Phe Pro Arg Ser Arg Leu Arg Phe Lys
                   565                 570                 575
   Glu Lys Leu Gly Glu Gly Gln Phe Gly Glu Val His Leu Cys Glu Val
               580                 585                 590
   Asp Ser Pro Gln Asp Leu Val Ser Leu Asp Phe Pro Leu Asn Val Arg
           595                 600                 605
   Lys Gly His Pro Leu Leu Val Ala Val Lys Ile Leu Arg Pro Asp Ala
       610                 615                 620
   Thr Lys Asn Ala Arg Asn Asp Phe Leu Lys Glu Val Lys Ile Met Ser
   625                 630                 635                 640
   Arg Leu Lys Asp Pro Asn Ile Ile Arg Leu Leu Gly Val Cys Val Gln
                   645                 650                 655
   Asp Asp Pro Leu Cys Met Ile Thr Asp Tyr Met Glu Asn Gly Asp Leu
               660                 665                 670
   Asn Gln Phe Leu Ser Ala His Gln Leu Glu Asp Lys Ala Ala Glu Gly
           675                 680                 685
   Ala Pro Gly Asp Gly Gln Ala Ala Gln Gly Pro Thr Ile Ser Tyr Pro
       690                 695                 700
   Met Leu Leu His Val Ala Ala Gln Ile Ala Ser Gly Met Arg Tyr Leu
   705                 710                 715                 720
   Ala Thr Leu Asn Phe Val His Arg Asp Leu Ala Thr Arg Asn Cys Leu
                   725                 730                 735
   Val Gly Glu Asn Phe Thr Ile Lys Ile Ala Asp Phe Gly Met Ser Arg
               740                 745                 750
   Asn Leu Tyr Ala Gly Asp Tyr Tyr Arg Val Gln Gly Arg Ala Val Leu
           755                 760                 765
   Pro Ile Arg Trp Met Ala Trp Glu Cys Ile Leu Met Gly Lys Phe Thr
       770                 775                 780
   Thr Ala Ser Asp Val Trp Ala Phe Gly Val Thr Leu Trp Glu Val Leu
   785                 790                 795                 800
   Met Leu Cys Arg Ala Gln Pro Phe Gly Gln Leu Thr Asp Glu Gln Val
                   805                 810                 815
   Ile Glu Asn Ala Gly Glu Phe Phe Arg Asp Gln Gly Arg Gln Val Tyr
               820                 825                 830
   Leu Ser Arg Pro Pro Ala Cys Pro Gln Gly Leu Tyr Glu Leu Met Leu
           835                 840                 845
   Arg Cys Trp Ser Arg Glu Ser Glu Gln Arg Pro Pro Phe Ser Gln Leu
       850                 855                 860
   His Arg Phe Leu Ala Glu Asp Ala Leu Asn Thr Val
   865                 870                 875

<210>  3
<211>  3877
<212>  DNA
<213>  Homo sapiens

<400>  3
tgctctcggg agccgcctcc cgacacccga gccccgccgg cgcctcccgc tcccggctcc      60

cggctcctgg ctccctccgc ctcccccgcc cctcgccccg ccgccgaaga ggccccgctc     120

ccgggtcgga cgcctgggtc tgccgggaag agcgatgaga ggtgtctgaa ggtggctatt     180

cactgagcga tggggttgga cttgaaggaa tgccaagaga tgctgccccc acccccttag     240

gcccgaggga tcaggagcta tgggaccaga ggccctgtca tctttactgc tgctgctctt     300

ggtggcaagt ggagatgctg acatgaaggg acattttgat cctgccaagt gccgctatgc     360

cctgggcatg caggaccgga ccatcccaga cagtgacatc tctgcttcca gctcctggtc     420

agattccact gccgcccgcc acagcaggtt ggagagcagt gacggggatg gggcctggtg     480

ccccgcaggg tcggtgtttc ccaaggagga ggagtacttg caggtggatc tacaacgact     540

gcacctggtg gctctggtgg gcacccaggg acggcatgcc gggggcctgg gcaaggagtt     600

ctcccggagc taccggctgc gttactcccg ggatggtcgc cgctggatgg gctggaagga     660

ccgctggggt caggaggtga tctcaggcaa tgaggaccct gagggagtgg tgctgaagga     720

ccttgggccc cccatggttg cccgactggt tcgcttctac ccccgggctg accgggtcat     780

gagcgtctgt ctgcgggtag agctctatgg ctgcctctgg agggatggac tcctgtctta     840

caccgcccct gtggggcaga caatgtattt atctgaggcc gtgtacctca acgactccac     900

ctatgacgga cataccgtgg gcggactgca gtatgggggt ctgggccagc tggcagatgg     960

tgtggtgggg ctggatgact ttaggaagag tcaggagctg cgggtctggc caggctatga 1020
ctatgtggga tggagcaacc acagcttctc cagtggctat gtggagatgg agtttgagtt 1080
tgaccggctg agggccttcc aggctatgca ggtccactgt aacaacatgc acacgctggg 1140
agcccgtctg cctggcgggg tggaatgtcg cttccggcgt ggccctgcca tggcctggga 1200
gggggagccc atgcgccaca acctaggggg caacctgggg gaccccagag cccgggctgt 1260
ctcagtgccc cttggcggcc gtgtggctcg ctttctgcag tgccgcttcc tctttgcggg 1320
gccctggtta ctcttcagcg aaatctcctt catctctgat gtggtgaaca attcctctcc 1380
ggcactggga ggcaccttcc cgccagcccc ctggtggccg cctggcccac ctcccaccaa 1440
cttcagcagc ttggagctgg agcccagagg ccagcagccc gtggccaagg ccgaggggag 1500
cccgaccgcc atcctcatcg gctgcctggt ggccatcatc ctgctcctgc tgctcatcat 1560
tgccctcatg ctctggcggc tgcactggcg caggctcctc agcaaggctg aacggagggt 1620
gttggaagag gagctgacgg ttcacctctc tgtccctggg gacactatcc tcatcaacaa 1680
ccgcccaggt cctagagagc cacccccgta ccaggagccc cggcctcgtg ggaatccgcc 1740
ccactccgct ccctgtgtcc ccaatggctc tgcgttgctg ctctccaatc cagcctaccg 1800
cctccttctg gccacttacg cccgtccccc tcgaggcccg ggccccccca cacccgcctg 1860
ggccaaaccc accaacaccc aggcctacag tggggactat atggagcctg agaagccagg 1920
cgccccgctt ctgcccccac ctccccagaa cagcgtcccc cattatgccg aggctgacat 1980
tgttaccctg cagggcgtca ccgggggcaa cacctatgct gtgcctgcac tgcccccagg 2040
ggcagtcggg gatgggcccc ccagagtgga tttccctcga tctcgactcc gcttcaagga 2100
gaagcttggc gagggccagt ttggggaggt gcacctgtgt gaggtcgaca gccctcaaga 2160
tctggttagt cttgatttcc cccttaatgt gcgtaaggga caccctttgc tggtagctgt 2220
caagatctta cggccagatg ccaccaagaa tgccaggaat gatttcctga aagaggtgaa 2280
gatcatgtcg aggctcaagg acccaaacat cattcggctg ctgggcgtgt gtgtgcagga 2340
cgaccccctc tgcatgatta ctgactacat ggagaacggc gacctcaacc agttcctcag 2400
tgcccaccag ctggaggaca aggcagccga gggggcccct ggggacgggc aggctgcgca 2460
ggggcccacc atcagctacc caatgctgct gcatgtggca gcccagatcg cctccggcat 2520
gcgctatctg gccacactca actttgtaca tcgggacctg gccacgcgga actgcctagt 2580
tggggaaaat ttcaccatca aaatcgcaga ctttggcatg agccggaacc tctatgctgg 2640
ggactattac cgtgtgcagg gccgggcagt gctgcccatc cgctggatgg cctgggagtg 2700
catcctcatg gggaagttca cgactgcgag tgacgtgtgg gcctttggtg tgaccctgtg 2760
ggaggtgctg atgctctgta gggcccagcc ctttgggcag ctcaccgacg agcaggtcat 2820
cgagaacgcg ggggagttct tccgggacca gggccggcag gtgtacctgt cccggccgcc 2880
tgcctgcccg cagggcctat atgagctgat gcttcggtgc tggagccggg agtctgagca 2940
gcgaccaccc ttttcccagc tgcatcggtt cctggcagag gatgcactca acacggtgtg 3000

aatcacacat ccagctgccc ctccctcagg gagcgatcca ggggaagcca gtgacactaa 3060

aacaagagga cacaatggca cctctgccct tcccctcccg acagcccatc acctctaata 3120

gaggcagtga gactgcaggt gggctgggcc cacccaggga gctgatgccc cttctcccct 3180

tcctggacac actctcatgt ccccttcctg ttcttccttc ctagaagccc ctgtcgccca 3240

cccagctggt cctgtggatg ggatcctctc caccctcctc tagccatccc ttggggaagg 3300

gtggggagaa atataggata gacactggac atggcccatt ggagcacctg ggccccactg 3360

gacaacactg attcctggag aggtggctgc gcccccagct tctctctccc tgtcacacac 3420

tggaccccac tggctgagaa tctgggggtg aggaggacaa gaaggagagg aaaatgtttc 3480

cttgtgcctg ctcctgtact tgtcctcagc ttgggcttct tcctcctcca tcacctgaaa 3540

cactggacct gggggtagcc ccgccccagc cctcagtcac ccccacttcc cacttgcagt 3600

cttgtagcta gaacttctct aagcctatac gtttctgtgg agtaaatatt gggattgggg 3660

ggaaagaggg agcaacggcc catagccttg gggttggaca tctctagtgt agctgccaca 3720

ttgatttttc tataatcact tggggtttgt acatttttgg ggggagagac acagattttt 3780

acactaatat atggacctag cttgaggcaa ttttaatccc ctgcactagg caggtaataa 3840

taaaggttga gttttccaca aaaaaaaaaa aaaaaaa 3877

<210> 4
<211> 913
<212> PRT
<213> Homo sapiens

<400> 4

Met Gly Pro Glu Ala Leu Ser Ser Leu Leu Leu Leu Leu Leu Val Ala
1               5                   10                  15

Ser Gly Asp Ala Asp Met Lys Gly His Phe Asp Pro Ala Lys Cys Arg
            20                  25                  30

Tyr Ala Leu Gly Met Gln Asp Arg Thr Ile Pro Asp Ser Asp Ile Ser
        35                  40                  45

Ala Ser Ser Ser Trp Ser Asp Ser Thr Ala Ala Arg His Ser Arg Leu
    50                  55                  60

Glu Ser Ser Asp Gly Asp Gly Ala Trp Cys Pro Ala Gly Ser Val Phe
65                  70                  75                  80

Pro Lys Glu Glu Glu Tyr Leu Gln Val Asp Leu Gln Arg Leu His Leu
                85                  90                  95

Val Ala Leu Val Gly Thr Gln Gly Arg His Ala Gly Gly Leu Gly Lys
            100                 105                 110

Glu Phe Ser Arg Ser Tyr Arg Leu Arg Tyr Ser Arg Asp Gly Arg Arg
        115                 120                 125

Trp Met Gly Trp Lys Asp Arg Trp Gly Gln Glu Val Ile Ser Gly Asn
    130                 135                 140

Glu Asp Pro Glu Gly Val Val Leu Lys Asp Leu Gly Pro Pro Met Val
145                 150                 155                 160

Ala Arg Leu Val Arg Phe Tyr Pro Arg Ala Asp Arg Val Met Ser Val
                165                 170                 175

Cys Leu Arg Val Glu Leu Tyr Gly Cys Leu Trp Arg Asp Gly Leu Leu
            180                 185                 190

Ser Tyr Thr Ala Pro Val Gly Gln Thr Met Tyr Leu Ser Glu Ala Val
        195                 200                 205

Tyr Leu Asn Asp Ser Thr Tyr Asp Gly His Thr Val Gly Gly Leu Gln
    210                 215                 220

Tyr Gly Gly Leu Gly Gln Leu Ala Asp Gly Val Val Gly Leu Asp Asp
225                 230                 235                 240

Phe Arg Lys Ser Gln Glu Leu Arg Val Trp Pro Gly Tyr Asp Tyr Val
                245                 250                 255

Gly Trp Ser Asn His Ser Phe Ser Ser Gly Tyr Val Glu Met Glu Phe
            260                 265                 270

Glu Phe Asp Arg Leu Arg Ala Phe Gln Ala Met Gln Val His Cys Asn
        275                 280                 285

Asn Met His Thr Leu Gly Ala Arg Leu Pro Gly Gly Val Glu Cys Arg
    290                 295                 300

Phe Arg Arg Gly Pro Ala Met Ala Trp Glu Gly Glu Pro Met Arg His
305                 310                 315                 320

Asn Leu Gly Gly Asn Leu Gly Asp Pro Arg Ala Arg Ala Val Ser Val
                325                 330                 335

Pro Leu Gly Gly Arg Val Ala Arg Phe Leu Gln Cys Arg Phe Leu Phe
            340                 345                 350

Ala Gly Pro Trp Leu Leu Phe Ser Glu Ile Ser Phe Ile Ser Asp Val
        355                 360                 365

Val Asn Asn Ser Ser Pro Ala Leu Gly Gly Thr Phe Pro Pro Ala Pro
    370                 375                 380

Trp Trp Pro Pro Gly Pro Pro Pro Thr Asn Phe Ser Ser Leu Glu Leu
385                 390                 395                 400

Glu Pro Arg Gly Gln Gln Pro Val Ala Lys Ala Glu Gly Ser Pro Thr
                405                 410                 415

Ala Ile Leu Ile Gly Cys Leu Val Ala Ile Ile Leu Leu Leu Leu Leu
            420                 425                 430

Ile Ile Ala Leu Met Leu Trp Arg Leu His Trp Arg Arg Leu Leu Ser
        435                 440                 445

Lys Ala Glu Arg Arg Val Leu Glu Glu Glu Leu Thr Val His Leu Ser
    450                 455                 460

Val Pro Gly Asp Thr Ile Leu Ile Asn Asn Arg Pro Gly Pro Arg Glu
465                 470                 475                 480

Pro Pro Pro Tyr Gln Glu Pro Arg Pro Arg Gly Asn Pro Pro His Ser
                485                 490                 495

Ala Pro Cys Val Pro Asn Gly Ser Ala Leu Leu Leu Ser Asn Pro Ala
            500                 505                 510

Tyr Arg Leu Leu Leu Ala Thr Tyr Ala Arg Pro Pro Arg Gly Pro Gly
        515                 520                 525

Pro Pro Thr Pro Ala Trp Ala Lys Pro Thr Asn Thr Gln Ala Tyr Ser
    530                 535                 540

Gly Asp Tyr Met Glu Pro Glu Lys Pro Gly Ala Pro Leu Leu Pro Pro
545                 550                 555                 560

Pro Pro Gln Asn Ser Val Pro His Tyr Ala Glu Ala Asp Ile Val Thr
                565                 570                 575

Leu Gln Gly Val Thr Gly Gly Asn Thr Tyr Ala Val Pro Ala Leu Pro
            580                 585                 590

Pro Gly Ala Val Gly Asp Gly Pro Pro Arg Val Asp Phe Pro Arg Ser
        595                 600                 605

Arg Leu Arg Phe Lys Glu Lys Leu Gly Glu Gly Gln Phe Gly Glu Val
    610                 615                 620

His Leu Cys Glu Val Asp Ser Pro Gln Asp Leu Val Ser Leu Asp Phe
625                 630                 635                 640

Pro Leu Asn Val Arg Lys Gly His Pro Leu Leu Val Ala Val Lys Ile
                645                 650                 655

Leu Arg Pro Asp Ala Thr Lys Asn Ala Arg Asn Asp Phe Leu Lys Glu
            660                 665                 670

Val Lys Ile Met Ser Arg Leu Lys Asp Pro Asn Ile Ile Arg Leu Leu

675                 680                 685

Gly Val Cys Val Gln Asp Asp Pro Leu Cys Met Ile Thr Asp Tyr Met
    690                 695                 700

Glu Asn Gly Asp Leu Asn Gln Phe Leu Ser Ala His Gln Leu Glu Asp
705                 710                 715                 720

Lys Ala Ala Glu Gly Ala Pro Gly Asp Gly Gln Ala Ala Gln Gly Pro
                725                 730                 735

Thr Ile Ser Tyr Pro Met Leu Leu His Val Ala Ala Gln Ile Ala Ser
            740                 745                 750

Gly Met Arg Tyr Leu Ala Thr Leu Asn Phe Val His Arg Asp Leu Ala
        755                 760                 765

Thr Arg Asn Cys Leu Val Gly Glu Asn Phe Thr Ile Lys Ile Ala Asp
    770                 775                 780

Phe Gly Met Ser Arg Asn Leu Tyr Ala Gly Asp Tyr Tyr Arg Val Gln
785                 790                 795                 800

Gly Arg Ala Val Leu Pro Ile Arg Trp Met Ala Trp Glu Cys Ile Leu
                805                 810                 815

Met Gly Lys Phe Thr Thr Ala Ser Asp Val Trp Ala Phe Gly Val Thr
            820                 825                 830

Leu Trp Glu Val Leu Met Leu Cys Arg Ala Gln Pro Phe Gly Gln Leu
        835                 840                 845

Thr Asp Glu Gln Val Ile Glu Asn Ala Gly Glu Phe Phe Arg Asp Gln
    850                 855                 860

Gly Arg Gln Val Tyr Leu Ser Arg Pro Pro Ala Cys Pro Gln Gly Leu
865                 870                 875                 880

Tyr Glu Leu Met Leu Arg Cys Trp Ser Arg Glu Ser Glu Gln Arg Pro
                885                 890                 895

Pro Phe Ser Gln Leu His Arg Phe Leu Ala Glu Asp Ala Leu Asn Thr
            900                 905                 910

Val

<210>  5
<211>  3678
<212>  DNA
<213>  Homo sapiens

<400>  5

agatgctgcc cccaccccct taggcccgag ggatcaggag ctatgggacc agaggccctg 60
tcatctttac tgctgctgct cttggtggca agtggagatg ctgacatgaa gggacatttt 120
gatcctgcca agtgccgcta tgccctgggc atgcaggacc ggaccatccc agacagtgac 180
atctctgctt ccagctcctg gtcagattcc actgccgccc gccacagcag gttggagagc 240
agtgacgggg atggggcctg gtgccccgca gggtcggtgt ttcccaagga ggaggagtac 300
ttgcaggtgg atctacaacg actgcacctg gtggctctgg tgggcaccca gggacggcat 360
gccgggggcc tgggcaagga gttctcccgg agctaccggc tgcgttactc ccgggatggt 420
cgccgctgga tgggctggaa ggaccgctgg ggtcaggagg tgatctcagg caatgaggac 480
cctgagggag tggtgctgaa ggaccttggg ccccccatgg ttgcccgact ggttcgcttc 540
tacccccggg ctgaccgggt catgagcgtc tgtctgcggg tagagctcta tggctgcctc 600
tggagggatg gactcctgtc ttacaccgcc cctgtggggc agacaatgta tttatctgag 660
gccgtgtacc tcaacgactc cacctatgac ggacataccg tgggcggact gcagtatggg 720
ggtctgggcc agctggcaga tggtgtggtg gggctggatg actttaggaa gagtcaggag 780
ctgcgggtct ggccaggcta tgactatgtg ggatggagca accacagctt ctccagtggc 840
tatgtggaga tggagtttga gtttgaccgg ctgagggcct tccaggctat gcaggtccac 900
tgtaacaaca tgcacacgct gggagcccgt ctgcctggcg gggtggaatg tcgcttccgg 960
cgtggccctg ccatggcctg ggagggggag cccatgcgcc acaacctagg gggcaacctg 1020
ggggacccca gagcccgggc tgtctcagtg ccccttggcg gccgtgtggc tcgctttctg 1080
cagtgccgct tcctctttgc ggggccctgg ttactcttca gcgaaatctc cttcatctct 1140
gatgtggtga acaattcctc tccggcactg ggaggcacct tcccgccagc cccctggtgg 1200
ccgcctggcc cacctcccac caacttcagc agcttggagc tggagcccag aggccagcag 1260
cccgtggcca aggccgaggg gagcccgacc gccatcctca tcggctgcct ggtggccatc 1320
atcctgctcc tgctgctcat cattgccctc atgctctggc ggctgcactg gcgcaggctc 1380
ctcagcaagg ctgaacggag ggtgttggaa gaggagctga cggttcacct ctctgtccct 1440
ggggacacta tcctcatcaa caaccgccca ggtcctagag agccaccccc gtaccaggag 1500
ccccggcctc gtgggaatcc gccccactcc gctccctgtg tccccaatgg ctctgcgttg 1560
ctgctctcca atccagccta ccgcctcctt ctggccactt acgcccgtcc ccctcgaggc 1620
ccgggccccc ccacacccgc ctgggccaaa cccaccaaca cccaggccta cagtggggac 1680
tatatggagc ctgagaagcc aggcgccccg cttctgcccc cacctcccca gaacagcgtc 1740
ccccattatg ccgaggctga cattgttacc ctgcagggcg tcaccggggg caacacctat 1800
gctgtgcctg cactgccccc aggggcagtc ggggatgggc cccccagagt ggatttccct 1860
cgatctcgac tccgcttcaa ggagaagctt ggcgagggcc agtttgggga ggtgcacctg 1920
tgtgaggtcg acagccctca agatctggtt agtcttgatt tcccccttaa tgtgcgtaag 1980
ggacaccctt tgctggtagc tgtcaagatc ttacggccag atgccaccaa gaatgccagc 2040
ttctccttgt tctccaggaa tgatttcctg aaagaggtga agatcatgtc gaggctcaag 2100

gacccaaaca tcattcggct gctgggcgtg tgtgtgcagg acgaccccct ctgcatgatt 2160

actgactaca tggagaacgg cgacctcaac cagttcctca gtgcccacca gctggaggac 2220

aaggcagccg agggggcccc tggggacggg caggctgcgc aggggcccac catcagctac 2280

ccaatgctgc tgcatgtggc agcccagatc gcctccggca tgcgctatct ggccacactc 2340

aactttgtac atcgggacct ggccacgcgg aactgcctag ttggggaaaa tttcaccatc 2400

aaaatcgcag actttggcat gagccggaac ctctatgctg gggactatta ccgtgtgcag 2460

ggccgggcag tgctgcccat ccgctggatg gcctgggagt gcatcctcat ggggaagttc 2520

acgactgcga gtgacgtgtg ggcctttggt gtgaccctgt gggaggtgct gatgctctgt 2580

agggcccagc cctttgggca gctcaccgac gagcaggtca tcgagaacgc gggggagttc 2640

ttccgggacc agggccggca ggtgtacctg tcccggccgc ctgcctgccc gcagggccta 2700

tatgagctga tgcttcggtg ctggagccgg gagtctgagc agcgaccacc cttttcccag 2760

ctgcatcggt tcctggcaga ggatgcactc aacacggtgt gaatcacaca tccagctgcc 2820

cctccctcag ggagcgatcc aggggaagcc agtgacacta aaacaagagg acacaatggc 2880

acctctgccc ttcccctccc gacagcccat cacctctaat agaggcagtg agactgcagg 2940

tgggctgggc ccacccaggg agctgatgcc ccttctcccc ttcctggaca cactctcatg 3000

tccccttcct gttcttcctt cctagaagcc cctgtcgccc acccagctgg tcctgtggat 3060

gggatcctct ccaccctcct ctagccatcc cttggggaag ggtggggaga aatataggat 3120

agacactgga catggcccat tggagcacct gggccccact ggacaacact gattcctgga 3180

gaggtggctg cgcccccagc ttctctctcc ctgtcacaca ctggacccca ctggctgaga 3240

atctgggggt gaggaggaca agaaggagag gaaaatgttt ccttgtgcct gctcctgtac 3300

ttgtcctcag cttgggcttc ttcctcctcc atcacctgaa acactggacc tgggggtagc 3360

cccgccccag ccctcagtca cccccacttc ccacttgcag tcttgtagct agaacttctc 3420

taagcctata cgtttctgtg gagtaaatat tgggattggg gggaaagagg gagcaacggc 3480

ccatagcctt ggggttggac atctctagtg tagctgccac attgattttt ctataatcac 3540

ttggggtttg tacatttttg gggggagaga cacagatttt tacactaata tatggaccta 3600

gcttgaggca attttaatcc cctgcactag gcaggtaata ataaaggttg agttttccac 3660

aaaaaaaaaa aaaaaaaa 3678

<210> 6
<211> 919
<212> PRT
<213> Homo sapiens

<400> 6

Met Gly Pro Glu Ala Leu Ser Ser Leu Leu Leu Leu Leu Leu Val Ala
1               5                   10                  15

Ser Gly Asp Ala Asp Met Lys Gly His Phe Asp Pro Ala Lys Cys Arg
            20                  25                  30

Tyr Ala Leu Gly Met Gln Asp Arg Thr Ile Pro Asp Ser Asp Ile Ser
        35                  40                  45

Ala Ser Ser Ser Trp Ser Asp Ser Thr Ala Ala Arg His Ser Arg Leu
    50                  55                  60

Glu Ser Ser Asp Gly Asp Gly Ala Trp Cys Pro Ala Gly Ser Val Phe
65                  70                  75                  80

Pro Lys Glu Glu Glu Tyr Leu Gln Val Asp Leu Gln Arg Leu His Leu
                85                  90                  95

Val Ala Leu Val Gly Thr Gln Gly Arg His Ala Gly Gly Leu Gly Lys
            100                 105                 110

Glu Phe Ser Arg Ser Tyr Arg Leu Arg Tyr Ser Arg Asp Gly Arg Arg
        115                 120                 125

Trp Met Gly Trp Lys Asp Arg Trp Gly Gln Glu Val Ile Ser Gly Asn
    130                 135                 140

Glu Asp Pro Glu Gly Val Val Leu Lys Asp Leu Gly Pro Pro Met Val
145                 150                 155                 160

Ala Arg Leu Val Arg Phe Tyr Pro Arg Ala Asp Arg Val Met Ser Val
                165                 170                 175

Cys Leu Arg Val Glu Leu Tyr Gly Cys Leu Trp Arg Asp Gly Leu Leu
            180                 185                 190

Ser Tyr Thr Ala Pro Val Gly Gln Thr Met Tyr Leu Ser Glu Ala Val
        195                 200                 205

Tyr Leu Asn Asp Ser Thr Tyr Asp Gly His Thr Val Gly Gly Leu Gln
    210                 215                 220

Tyr Gly Gly Leu Gly Gln Leu Ala Asp Gly Val Val Gly Leu Asp Asp
225                 230                 235                 240

Phe Arg Lys Ser Gln Glu Leu Arg Val Trp Pro Gly Tyr Asp Tyr Val
                245                 250                 255

Gly Trp Ser Asn His Ser Phe Ser Ser Gly Tyr Val Glu Met Glu Phe
            260                 265                 270

Glu Phe Asp Arg Leu Arg Ala Phe Gln Ala Met Gln Val His Cys Asn
        275                 280                 285

Asn Met His Thr Leu Gly Ala Arg Leu Pro Gly Gly Val Glu Cys Arg
    290                 295                 300

Phe Arg Arg Gly Pro Ala Met Ala Trp Glu Gly Glu Pro Met Arg His
305                 310                 315                 320

Asn Leu Gly Gly Asn Leu Gly Asp Pro Arg Ala Arg Ala Val Ser Val
                325                 330                 335

Pro Leu Gly Gly Arg Val Ala Arg Phe Leu Gln Cys Arg Phe Leu Phe
            340                 345                 350

Ala Gly Pro Trp Leu Leu Phe Ser Glu Ile Ser Phe Ile Ser Asp Val
        355                 360                 365

Val Asn Asn Ser Ser Pro Ala Leu Gly Gly Thr Phe Pro Pro Ala Pro
    370                 375                 380

Trp Trp Pro Pro Gly Pro Pro Pro Thr Asn Phe Ser Ser Leu Glu Leu
385                 390                 395                 400

Glu Pro Arg Gly Gln Gln Pro Val Ala Lys Ala Glu Gly Ser Pro Thr
                405                 410                 415

Ala Ile Leu Ile Gly Cys Leu Val Ala Ile Ile Leu Leu Leu Leu
            420                 425                 430

Ile Ile Ala Leu Met Leu Trp Arg Leu His Trp Arg Arg Leu Leu Ser
        435                 440                 445

Lys Ala Glu Arg Arg Val Leu Glu Glu Glu Leu Thr Val His Leu Ser
    450                 455                 460

Val Pro Gly Asp Thr Ile Leu Ile Asn Asn Arg Pro Gly Pro Arg Glu
465                 470                 475                 480

Pro Pro Pro Tyr Gln Glu Pro Arg Pro Arg Gly Asn Pro Pro His Ser
                485                 490                 495

Ala Pro Cys Val Pro Asn Gly Ser Ala Leu Leu Leu Ser Asn Pro Ala
            500                 505                 510

Tyr Arg Leu Leu Leu Ala Thr Tyr Ala Arg Pro Pro Arg Gly Pro Gly
        515                 520                 525

Pro Pro Thr Pro Ala Trp Ala Lys Pro Thr Asn Thr Gln Ala Tyr Ser
    530                 535                 540

Gly Asp Tyr Met Glu Pro Glu Lys Pro Gly Ala Pro Leu Leu Pro Pro
545                 550                 555                 560

Pro Pro Gln Asn Ser Val Pro His Tyr Ala Glu Ala Asp Ile Val Thr
                565                 570                 575

Leu Gln Gly Val Thr Gly Gly Asn Thr Tyr Ala Val Pro Ala Leu Pro
            580                 585                 590

Pro Gly Ala Val Gly Asp Gly Pro Pro Arg Val Asp Phe Pro Arg Ser
        595                 600                 605

Arg Leu Arg Phe Lys Glu Lys Leu Gly Glu Gly Gln Phe Gly Glu Val
    610                 615                 620

His Leu Cys Glu Val Asp Ser Pro Gln Asp Leu Val Ser Leu Asp Phe
625                 630                 635                 640

Pro Leu Asn Val Arg Lys Gly His Pro Leu Leu Val Ala Val Lys Ile
                645                 650                 655

Leu Arg Pro Asp Ala Thr Lys Asn Ala Ser Phe Ser Leu Phe Ser Arg
            660                 665                 670

Asn Asp Phe Leu Lys Glu Val Lys Ile Met Ser Arg Leu Lys Asp Pro
        675                 680                 685

Asn Ile Ile Arg Leu Leu Gly Val Cys Val Gln Asp Asp Pro Leu Cys
    690                 695                 700


705                 710                 715                 720


                725                 730                 735


            740                 745                 750


        755                 760                 765


    770                 775                 780


785                 790                 795                 800


                805                 810                 815


            820                 825                 830


        835                 840                 845



850                 855                 860


865                 870                 875                 880


                885                 890                 895


            900                 905                 910


        915

<210>  7
<211>  3252
<212>  DNA
<213>  Homo sapiens

<400>  7
gaatcctcag aatgtgaggt ttctctcaaa ggcatcttgc atcagcctgt ggatgtatgc      60

ctaccaccgg gctccttcac cagcaaagtg gaaaaagaag cgtttcacaa caaattcttc     120

tttttgggtt ggggaaacgc agtggattat agctctgttt tcttctttcc aaaactgtgc     180

acccctggat gaaacctcca tcaagggaga cctacaagtt gcctggggtt cagtgctcta     240

gaaagttcca aggtttgtgg cttgaattat tctaaagaag ctgaaataat tgaagagaag     300

cagaggccag ctgtttttga ggatcctgct ccacagagaa tgctctgcac ccgttgatat     360

gcctcccagg acccagaggg agactgtagc ctcatttctg tggagacctt tggctggact     420

ctcctggctc tcccagagac tccagttcca acaccatctt ctgagatgat cctgattccc     480

agaatgctct tggtgctgtt cctgctgctg cctatcttga gttctgcaaa agctcaggtt     540

aatccagcta tatgccgcta tcctctgggc atgtcaggag gccagattcc agatgaggac     600

atcacagctt ccagtcagtg gtcagagtcc acagctgcca aatatggaag gctggactca     660

gaagaagggg atggagcctg gtgccctgag attccagtgg aacctgatga cctgaaggag     720

tttctgcaga ttgacttgca caccctccat tttatcactc tggtggggac ccaggggcgc     780

catgcaggag gtcatggcat cgagtttgcc cccatgtaca agatcaatta cagtcgggat     840

ggcactcgct ggatctcttg gcggaaccgt catgggaaac aggtgctgga tggaaatagt     900

aacccctatg acattttcct aaaggacttg gagccgccca ttgtagccag atttgtccgg     960

ttcattccag tcaccgacca ctccatgaat gtgtgtatga gagtggagct ttacggctgt    1020

gtctggctag atggcttggt gtcttacaat gctccagctg ggcagcagtt tgtactccct    1080

ggaggttcca tcatttatct gaatgattct gtctatgatg gagctgttgg atacagcatg    1140

acagaagggc taggccaatt gaccgatggt gtgtctggcc tggacgattt cacccagacc    1200

catgaatacc acgtgtggcc cggctatgac tatgtgggct ggcggaacga gagtgccacc    1260

aatggctaca ttgagatcat gtttgaattt gaccgcatca ggaatttcac taccatgaag    1320

gtccactgca acaacatgtt tgctaaaggt gtgaagatct ttaaggaggt acagtgctac 1380
ttccgctctg aagccagtga gtgggaacct aatgccattt ccttccccct tgtcctggat 1440
gacgtcaacc ccagtgctcg gtttgtcacg gtgcctctcc accaccgaat ggccagtgcc 1500
atcaagtgtc aataccattt tgcagatacc tggatgatgt tcagtgagat caccttccaa 1560
tcagatgctg caatgtacaa caactctgaa gccctgccca cctctcctat ggcacccaca 1620
acctatgatc caatgcttaa agttgatgac agcaacactc ggatcctgat tggctgcttg 1680
gtggccatca tctttatcct cctggccatc attgtcatca tcctctggag gcagttctgg 1740
cagaaaatgc tggagaaggc ttctcggagg atgctggatg atgaaatgac agtcagcctt 1800
tccctgccaa gtgattctag catgttcaac aataaccgct cctcatcacc tagtgaacaa 1860
gggtccaact cgacttacga tcgcatcttt ccccttcgcc ctgactacca ggagccatcc 1920
aggctgatac gaaaactccc agaatttgct ccaggggagg aggagtcagg ctgcagcggt 1980
gttgtgaagc cagtccagcc cagtggccct gagggggtgc cccactatgc agaggctgac 2040
atagtgaacc tccaaggagt gacaggaggc aacacatact cagtgcctgc cgtcaccatg 2100
gacctgctct caggaaaaga tgtggctgtg gaggagttcc ccaggaaact cctaactttc 2160
aaagagaagc tgggagaagg acagtttggg gaggttcatc tctgtgaagt ggagggaatg 2220
gaaaaattca aagacaaaga ttttgcccta gatgtcagtg ccaaccagcc tgtcctggtg 2280
gctgtgaaaa tgctccgagc agatgccaac aagaatgcca ggaatgattt tcttaaggag 2340
ataaagatca tgtctcggct caaggaccca aacatcatcc atctattagc tgtgtgtatc 2400
actgatgacc ctctctgtat gatcactgaa tacatggaga atggagatct caatcagttt 2460
ctttcccgcc acgagccccc taattcttcc tccagcgatg tacgcactgt cagttacacc 2520
aatctgaagt ttatggctac ccaaattgcc tctggcatga agtacctttc ctctcttaat 2580
tttgttcacc gagatctggc cacacgaaac tgtttagtgg gtaagaacta cacaatcaag 2640
atagctgact ttggaatgag caggaacctg tacagtggtg actattaccg gatccagggc 2700
cgggcagtgc tccctatccg ctggatgtct tgggagagta tcttgctggg caagttcact 2760
acagcaagtg atgtgtgggc ctttggggtt actttgtggg agactttcac cttttgtcaa 2820
gaacagccct attcccagct gtcagatgaa caggttattg agaatactgg agagttcttc 2880
cgagaccaag ggaggcagac ttacctccct caaccagcca tttgtcctga ctctgtgtat 2940
aagctgatgc tcagctgctg gagaagagat acgaagaacc gtccctcatt ccaagaaatc 3000
caccttctgc tccttcaaca aggcgacgag tgatgctgtc agtgcctggc catgttccta 3060
cggctcaggt cctccctaca agacctacca ctcacccatg cctatgccac tccatctgga 3120
catttaatga aactgagaga cagaggcttg tttgctttgc cctcttttcc tggtcacccc 3180
cactccctac ccctgactca tatatacttt tttttttttt tacattaaag aactaaaaaa 3240
ggaaaaaaaa aa 3252

<210> 8
<211> 855

<212>  PRT
<213>  Homo sapiens

<400>  8